(19) Europäisches Patentamt
European Patent Office
Office européen des brevets

(11) Publication number: **0 057 401 B1**

(12) **EUROPEAN PATENT SPECIFICATION**

(45) Date of publication of the patent specification: **01.08.84**

(51) Int. Cl.³: **C 07 J 17/00**, C 07 J 33/00, C 07 J 43/00, A 61 K 31/58

(21) Application number: **82100490.0**

(22) Date of filing: **25.01.82**

(54) **Aromatic heterocyclic esters of steroids, their preparation and pharmaceutical compositions containing them.**

(30) Priority: **02.02.81 US 230763**

(43) Date of publication of application: **11.08.82 Bulletin 82/32**

(45) Publication of the grant of the patent: **01.08.84 Bulletin 84/31**

(84) Designated Contracting States: **AT BE CH DE FR GB IT LI NL SE**

(56) References cited:
**DE - A - 2 031 205**
**DE - B - 1 059 906**
**GB - A - 1 191 965**
**US - A - 4 221 787**

**ARZNEIMITTEL FORSCHUNG, vol. 29, no. 11, november 1979 AULENDORF (DE) B.N. LUTSKY et al.: "A novel class of potent topical antiinflammatory agents: 17-benzoylated, 7-halogeno substituted corticosteroids" pages 1662-1667**

(73) Proprietor: **SCHERING CORPORATION, 2000 Galloping Hill Road, Kenilworth, New Jersey 07033 (US)**

(72) Inventor: **Shapiro, Elliot L., 24 Brookshire Drive, Cedar Grove New Jersey 07009 (US)**

(74) Representative: **Antony, Fritz, Dr. et al, P.O. Box 601 Winkelriedstrasse 35, CH-6002 Lucerne (CH)**

**EP 0 057 401 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel aromatic heterocyclic esters of steroids, to their preparation and to pharmaceutical compositions containing them.

More specifically, this invention relates to novel 3,20-dioxo-1,4-pregnadiene-17α-ol 17-aromatic heterocyclic carboxylates, and the 1,2-dihydro and 6-dehydro derivatives thereof, useful in the treatment of inflammatory conditions.

The novel 17-aromatic heterocyclic carboxylates of this invention are 3,20-dioxo-1,4-pregnadienes of the following Formula I:

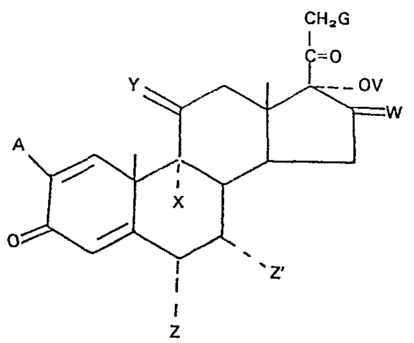

(I)

wherein:

A is hydrogen or, provided that Y is (H,β-OH), A may also be chloro, fluoro or methyl;

X is hydrogen or a halogen atom having an atomic weight less than 100;

Y is oxygen, (H,β-OH) or, provided that X is hydrogen, Y may also be (H,H) or, provided that X is chloro or bromo, Y may also be (H,β-halogen), the β-halogen having an atomic weight of less than 100, and being at least as electronegative as X;

Z is hydrogen, $CH_3$, chloro or fluoro, and Z' is hydrogen or, provided that Z is hydrogen, Z' may also be a halogen atom having an atomic weight less than 100;

V is an acyl radical of thiophenecarboxylic acid, pyrrolecarboxylic acid or furancarboxylic acid or the methyl- or halogen-substituted derivatives thereof;

W is (H,H), (H, lower alkyl) or (H,α$OV_1$) where $V_1$ is hydrogen or an acyl radical of retinoic acid or a carboxylic acid having up to 12 carbon atoms; or W is =CHT where T is hydrogen, lower alkyl, fluoro or chloro, and

G is hydrogen, a halogen atom having an atomic weight less than 100, or $QV_2$ where Q is an oxygen or sulfur atom and $V_2$ is as defined for V or $V_1$ or is an acyl radical of phosphoric acid which may be in the form of a mono- or dialkali metal or an alkaline earth metal salt thereof;

and the 6-dehydro and 1,2-dihydro derivatives of the foregoing.

The term lower alkyl as used herein means such groups having up to 6 carbon atoms and includes straight- and branched-chain groups. Examples include methyl, ethyl, n-propyl, isopropyl, n-butyl, sec.-butyl, n-pentyl, isopentyl, n-hexyl, and 2,3-dimethylbutyl.

The acyl radicals of the aromatic heterocyclic carboxylic acids specified in the definition of V in Formula I are those derived from 2-furancarboxylic acid, 3-furancarboxylic acid, 2-thiophenecarboxylic acid, 3-thiophenecarboxylic acid, 2-pyrrolecarboxylic acid, 3-pyrrolecarboxylic acid, and the methyl- and halogen-substituted derivatives thereof such as 5-methyl-2-thiophenecarboxylic acid, N-methyl-2-pyrrolecarboxylic acid, and 5-bromo-2-furancarboxylic acid. Preferred 17-esters are the 17-furoyl and 17-thenoyl esters.

The acyl radicals specified in the definition of $V_1$ in Formula I are those derived from carboxylic acids having up to 12 carbon atoms, which acids may be saturated, unsaturated, straight-chain or branched-chain, aliphatic, cyclic, cyclic-aliphatic, aromatic, aromatic heterocyclic, aryl-aliphatic, or alkyl-aromatic, and may be substituted by hydroxy or by alkoxy or alkylthio containing from 1 to 5 carbon atoms or by a halogen. Exemplary of such acyl radicals are those derived from alkanoic acids exemplified by formic, acetic, propionic, trimethylacetic, butyric, isobutyric, valeric, isovaleric, caproic, tert.-butylacetic, enanthic, caprylic, capric, cyclopentylpropionic, undecylic, lauric, and adamantanecarboxylic acids; substituted alkanoic acids such as phenoxyacetic, trifluoroacetic, and β-chloropropionic acids; aromatic and substituted aromatic acids, especially benzoic acids substituted by a halogen atom or a methoxy group, such as benzoic, toluic, p-chlorobenzoic, p-fluorobenzoic, p-methoxybenzoic, and 3',5'-dimethylbenzoic acids; aromatic-heterocyclic acids in particular isonicotinic acid; aryl-alkanoic acids such as phenylacetic, phenylpropionic, and β-benzoylaminoisobutyric acids; unsaturated acids such as acrylic and sorbic acids; and dibasic acids such as succinic, tartaric, phthalic and benzene disulfonic acids.

The preferred acyl groups are those derived from lower alkanoic acids having up to 8 carbon atoms, in particular from acetic, propionic, butyric, valeric, caprylic, caproic and t-butylacetic acids, and from benzoic acid or the substituted benzoic acids mentioned above as well as the corresponding C-1-5 alkoxy and alkylthio derivatives of the foregoing acyl groups in particular methoxyacetyl and methylthioacetyl.

Preferred 21-O-ester functions are the 21-acetate and the 21-methoxyacetate and preferred 21-thiol-21-esters are the 21-thiol lower alkanoates and lower alkoxyalkanoates, in particular the 21-thiol-21-pivalate.

The alkylidene groups represented by =CHT are preferably lower alkylidenes, i.e. hydrocarbon radicals having preferably up to 4 carbon atoms including radicals such as methylene, ethylidene, n-propylidene, isopropylidene, n-butylidene, and sec.-butylidene.

The 3,20-dioxo-1,4-pregnadiene-17α-ol 17-aromatic heterocyclic carboxylates of Formula I are usually white to off-white crystalline solids, which are insoluble in water (with the exception of alkali metal salts of esters such as the hemisuccinate and phosphate esters thereof) and soluble

in most organic solvents, particularly in acetone, dioxan, dimethylformamide, and dimethylsulfoxide, although of limited solubility in non-polar solvents such as dialkyl ethers and aliphatic hydrocarbons.

In general, the 3,20-dioxo-1,4-pregnadiene-17α-ol-17-aromatic heterocyclic carboxylates of Formula I, particularly those wherein G is $QV_2$ where $V_2$ is hydrogen or an acyl group as previously defined, exhibit corticosteroid activity. Those that have halogens at both C-9 and C-11 or an oxygen or β-hydroxyl function at C-11 and a halogen or hydrogen at C-9 possess glucocorticoid activity and are particularly valuable as anti-inflammatory agents.

Particularly useful topical anti-inflammatory agents are the 17-furoyl and 17-thenoyl esters of Formula I wherein G is halogeno or $QV_2$, and C-2 and C-7 are unsubstituted, especially those compounds substituted at C-16 by a lower alkyl group (particularly a 16-methyl group, e.g. 16α-methyl) which generally exhibit topical anti-inflammatory activity superior to the topical anti-inflammatory activity of the 17-non-heterocyclic-carboxylate derivatives corresponding to Formula I.

The 17-acyloxy-21-desoxyderivatives of Formula I, while exhibiting anti-inflammatory activity, are generally more valuable as progestational agents. A preferred group of 3,20-dioxo-1,4-pregnadiene-17α-ol 17-aromatic heterocyclic carboxylates exhibiting particularly useful anti-inflammatory activity can be represented by the general Formula II:

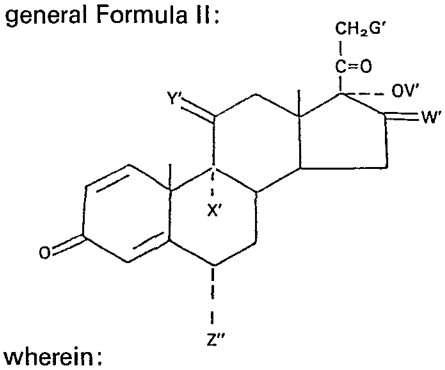

(II)

wherein:

X′ is fluoro or chloro;

Y′ is (H,β-OH) or, provided that X′ is chloro, Y′ may also be (H,β-halogen), the β-halogen having an atomic weight of less than 100 and being at least as electronegative as X′;

Z″ is hydrogen or fluoro;

W′ is (H, H) or (H, $CH_3$);

V′ is furancarbonyl or thiophenecarbonyl, and

G′ is chloro or fluoro, or $QV'_2$, wherein Q is sulfur or preferably oxygen and $V'_2$ is hydrogen or an acyl radical of retinoic acid, of carboxylic acids having up to 12 (preferably up to 8) carbon atoms or of phosphoric acid and which may be in the form of a mono- or dialkali metal or alkaline earth metal salt.

Preferred compounds include the 17-(2′-furoate), 17-(3′-furoate), 17-(2′-thenoate) and the 17-(3′-thenoate) ester derivatives of the following:

9α,11β,21-trichloro-16α-methyl-1,4-pregnadiene-17α-ol-3,20-dione;

9α,11β-dichloro-16α-methyl-1,4-pregnadiene-17α,21-diol-3,20-dione 21-acetate;

9α-chloro- (and the corresponding 9α-fluoro-) 16α-methyl-1,4-pregnadiene-11β,17α,21-triol-3,20-dione 21-acetate;

9α-chloro- (and the corresponding 9α-fluoro-) 21-chloro-16α-methyl-1,4-pregnadiene-11β, 17α-diol-3,20-dione;

as well as the following 21-analogs of the foregoing: 21-fluoro-, 21-methoxyacetate and 21-thio-21-pivalate. The 6α-fluoro derivatives of the foregoing also represent a preferred area of the compounds of the general Formula I, of which may be mentioned:

6α-fluoro-9α,11β-dichloro-16α-methyl-1,4-pregnadiene-17α,21-diol-3,20-dione 17-(2′-furoate) 21-acetate, and

6α-fluoro-9α,11β,21-trichloro-16α-methyl-1,4-pregnadiene-17α-ol-3,20-dione 17-(2′-furoate).

Other compounds which may be specifically referred to are the 16-unsubstituted analogs of the foregoing compounds as well as the 16β-methyl epimers thereof.

Of the foregoing, particularly valuable are 9α, 11β, 21-trichloro-16α-methyl-1,4-pregnadiene-17α-ol-3,20-dione 17-(2′-furoate) and 9α,21-dichloro-16α-methyl-1,4-pregnadiene-11β,17α-diol-3,20-dione 17-(2′-furoate) and 9α-fluoro-16α-methyl-1,4-pregnadiene-11β, 17α,21-triol-3,20-dione 17-(2′-furoate) which have high topical and local anti-inflammatory activity.

Further compounds of the Formula I which may be mentioned are:

1,4-pregnadiene-11β,17α,21-triol-3,20-dione 17-(2′-furoate) 21-acetate,

16α-methyl-1,4-pregnadiene-11β,17α,21-triol-3,20-dione 17-(2′-furoate) 21-acetate,

1,4,6-pregnatriene-11β,17α,21-triol-3,20-dione 17-(2′-furoate) 21-acetate,

4-pregnene-11β,17α,21-triol-3,20-dione 17-(2′-furoate) 21-acetate,

16α-methyl-1,4-pregnadiene-17α,21-diol-3,20-dione 17-(2′-furoate) 21-acetate, and

7α-chloro-16α-methyl-1,4-pregnadiene-11β, 17α,21-triol-3,20-dione 17-(2′-furoate) 21-acetate;

21-desoxy pregnadienes (i.e. compounds of Formula I wherein G is hydrogen) such as:

9α,11β-dichloro-16α-methyl-1,4-pregnadiene-17α-ol-3,20-dione 17-(2′-furoate),

9α-fluoro-16α-methyl-1,4-pregnadiene-11β, 17α-diol-3,20-dione 17-(2′-furoate),

9α-fluoro-16-methylene-1,4-pregnadiene-11β,17α,21-triol-3,20-dione 17-(2′-furoate) 21-acetate, and

9α-fluoro-1,4-pregnadiene-11β,16α,17α, 21-tetrol-3,20-dione 17-(2′-furoate) 16,21-diacetate.

The present invention also provides a process for the preparation of the 17α-aromatic heterocyclic carboxylates of the invention, which process

comprises subjecting an appropriate 3,20-dioxo-1,4-pregnadiene starting material, or a 6-dehydro or 1,2-dihydro derivative thereof, to one or more of the following general methods A to D, namely:

A: introduction of the desired ester group at the 16α,17α and/or 21 positions, or

B: hydrolysis of an ester group present at one or more of positions 11, 16α and 21 or of a 16α, 17α- or 17α,21 - orthoester group, or

C: halogenation at one or more of positions 9α,11β and 21, or

D: reduction of an 11-oxo group to an 11β-hydroxy group.

*General Method A*

The 17α-aromatic heterocyclic carboxylates of the present invention may conveniently be prepared by a process of general method A above, the process comprising esterification of a corresponding 17α-ol starting material having present in the molecule the other desired substituents. This method is particularly applicable to the preparation of those preferred compounds of the Formula I where X is chloro and Y is (H,β-Cl) or where X is fluoro and Y is (H,β-OH) and G is an acyloxy group as specified in the definition of $-QV_2$; that is for the preparation of specified 17α,21-diesters of the general Formula I.

Typically, reaction is effected by esterifying a 17α-hydroxy-21-acylate starting material with the required aromatic heterocyclic carboxylic acid, usually in the form of a reactive derivative of the acid such as an acid halide (e.g. acid chloride) or acid anhydride in the presence of a basic catalyst preferably a 4-dialkylaminopyridine such as 4-dimethylaminopyridine.

The 17α-esterification is preferably carried out under conditions which minimize undesirable side-reactions, such as hydrolysis of other ester functions present in the molecule. Thus, the use of aqueous or alcoholic media is generally to be avoided, the reaction being preferably effected in a non-reactive organic solvent under anhydrous conditions. Examples of suitable non-reactive organic solvents are acetonitrile, tetrahydrofuran, pyridine, dimethylformamide and, as a preferred solvent, methylene chloride. While esterification may be effected at any convenient temperature, it is preferred to use room temperature, that is about 20° C. The reaction is usually complete in 24 to 120 h depending upon the nature of the reactants and the reaction conditions used.

Prior to esterification of the 17α-hydroxy group, any desired free 11β-hydroxy (or 16α-hydroxy) function present in the molecule of the starting material is desirably protected in known manner with a suitable protecting group which, after esterification at C-17, is then removed.

17α,21-diesters falling within the scope of the present invention may alternatively be prepared by 21-acylation of a corresponding 21-hydroxy-17α-ester starting material with the appropriate acid, usually in the form of a reactive derivative, such as the acid anhydride or acid chloride, and preferably in the presence of a tertiary base such as pyridine. Any desired free hydroxy group at C-11 or C-16 may be suitably protected as mentioned above.

A 21-dihydrogenphosphate ester may be prepared by reaction of the corresponding 21-hydroxy compound with pyrophosphorylchloride. The mono- and dialkali metal salts and alkaline earth metal salts of the dihydrogen phosphate ester may be obtained by partial or complete neutralisation with an alkali metal methoxide or alkaline earth metal methoxide.

Esterification at C-17 is also a convenient method for preparing those compounds of the invention where X is hydrogen, Y is an oxygen atom and G is an acyloxy group as specified in the definition of $-QV_2$. Following esterification at C-17, the 11-oxo group may, if desired, be reduced to give further an 11β,17α,21-triol 17,21-diacylate of the present invention.

Those compounds of the invention where W in position 16 of Formula I is (H, O-acyl) as specified in the definition of $-OV_1$, that is 16α-esters of the general Formula I, may be prepared by esterification of a corresponding 16α-hydroxy group in a similar manner to that specified for esterification at C-21, any free hydroxy group at C-11 or C-21 being protected as necessary.

*General Method B*

17α-acyloxy-21-hydroxy compounds of the present invention may be prepared by a process of general method B above, the process comprising 21-deacylation of a corresponding 17α,21-ester. The hydrolysis step is typically effected under acid conditions using strong mineral acid, preferably 70% perchloric acid in methanol. Where it is desired to deacylate an 11β- and/or 16α-ester function present in the molecule of the starting material, this may similarly be effected using appropriate hydrolysis conditions known in the art.

17α-acyloxy-21-hydroxy compounds of the invention, as well as 17α-acyloxy-16α-hydroxy compounds, may also be prepared by hydrolysis of a corresponding 17α,21-orthoester or 16α,17α-orthoester respectively. The hydrolysis may be effected under mildly acidic conditions, e.g. in the presence of a lower alkanoic acid (e.g. acetic or propionic acid) or a strong mineral acid (e.g. hydrochloric or sulphuric acid).

The orthoester starting materials may be prepared in known manner by reacting the appropriate diol, for instance the corresponding 17α,21-diol, with a trialkylheterocyclic orthoester, such as 2-trimethylorthothenoate, 2-trimethylorthofuroate or 2-trimethylorthopyrroloate, in an appropriate organic solvent (e.g. a dioxan/benzene mixture) in the presence of a catalyst (e.g. pyridinium p-toluene sulfonate).

General method B also encompasses the hydrolysis, as a last step, of an 11β- and/or 16α-protected hydroxy group using appropriate hydrolysis conditions known in the art to give the desired compound of the Formula I. The method may suitably be used for the preparation of 17α,21-diesters of the general Formula I.

*General Method C*

A further general method for the preparation of the compounds of the invention, designated above as method C, comprises halogenation at one or more of positions 9α,11β and 21. This method is particularly applicable to the preparation of those preferred compounds of the general Formula I where G is halogen, in particular chloro or fluoro, or an acyloxy group as specified in the definition of $-QV_2$, and X is chloro and Y is (H,β-Cl) or X is chloro or fluoro and Y is (H,β-OH).

Thus, where a 9α,11β-dichloro compound of the general Formula I is required a 9(11)-dehydro-starting material bearing all the other desired structural variants may be treated with chlorine in a halogenated solvent (e.g. carbon tetrachloride) in the presence of a tertiary amine such as pyridine.

For the preparation of a 21-halogeno compound of the invention, a corresponding 21-sulfonate, such as a 21-mesylate or 21-tosylate, or a comparable ester, may be treated with a suitable source of the desired halogen ion, for instance with a tetraalkyl ammonium halide (e.g. chloride or fluoride) or alkali metal halide preferably with lithiumchloride where a 21-chloro compound is required. The reaction may typically be effected by heating the reactants in a suitable solvent, for instance dimethylformamide. This method is primarily applicable to those compounds of the Formula I where X is fluoro and Y is (H,β-OH) or where X is chloro and Y is (H,β-OH).

9α,11β,21-trihalogeno compounds of the invention are preferably prepared by 21-halogenation followed by 9α,11β-halogenation.

9α-halogeno-11β-hydroxy compounds, in particular 9α-chloro-11β-hydroxy compounds, of the invention may be prepared from a corresponding 9(11)-dehydro-starting material by reaction with a suitable halogenating agent such as an N-chloroamide, preferably 1,3-dichloro-5,5-dimethylhydantoin, and a strong mineral acid, preferably perchloric acid, in an inert organic solvent such as moist dioxan or tetrahydrofuran.

Alternatively, the 9α-halogeno-11β-hydroxy compounds of the general Formula I may be prepared by treating a corresponding 9β,11β-oxido-starting compound with hydrogen chloride or hydrogen fluoride in a suitable inert solvent. Thus, for instance, the 9α-chloro-11β-hydroxy compounds of the general Formula I (which represent a preferred group of compounds) may be prepared by treating a corresponding 9β,11β-oxido compound, preferably at room temperature, with anhydrous hydrogen chloride in a suitable inert medium, such as glacial acetic acid.

21-halogenation with concomitant introduction of the desired 17-aromatic heterocyclic ester group may be achieved by treating a 17α,21-aromatic heterocyclic orthoester with a triarylsilyl halide (such as a tritolylsilyl or triphenylsilyl halide) or a tri-lower alkylsilyl halide (such as a trimethylsilyl halide) in known manner as described, for instance in USP No. 3992422.

*General Method D*

A further general method for the preparation of 11β-hydroxy-17α-aromatic heterocyclic carboxylates of the present invention comprises reduction at the 11 position of a corresponding 11-oxo-17α-ester. This method is particularly applicable to the preparation of 11β-hydroxy-17α-aromatic heterocyclic carboxylate 21-acylates of the invention. Reduction may be effected in known manner, typically by treating an 11-oxo-17α,21-diester starting material with a suitable reducing agent including sodium, potassium or lithium borohydride, tetra-n-butylammonium borohydride or lithium tri-t-butoxyaluminium hydride, in an inert solvent, preferably sodium borohydride in an inert organic solvent such as dimethylformamide. Usually, reduction will be effected at somewhat reduced temperatures at or about 0° C.

The foregoing methods A to D are illustrated in the following specific examples, primarily by reference to the preparation of 3,20-dioxo-1,4-pregnadienes of the present invention. By use of the appropriate starting materials, however, the corresponding 3,20-dioxo-4-pregnenes and 3,20-dioxo-1,4,6-pregnatrienes may similarly be prepared. In preparing, by the foregoing methods A to D, the compounds of the Formula I bearing a substituent at one or more of positions 2,6,7 and 16, that substituent may conveniently be present in the starting material employed in the foregoing methods.

*Example 1*

*9α, 11β-dichloro-16-methyl-1,4-pregnadiene-17α, 21-diol-3,20-dione 17-heterocyclic carboxylate 21-alkanoates*

A) *9α,11β-dichloro-16α-methyl-1,4-pregnadiene-17α,21-diol-3,20-dione 17-(2'-furoate) 21-acetate*

Dissolve 4-dimethylaminopyridine (12 g), 9α,11β-dichloro-16α-methyl-1,4-pregnadiene-17α,21-diol-3,20-dione 21-acetate (4.8 g) and 2-furoylchloride (2 ml) in methylenechloride (62 ml) and stir at room temperature until thin-layer chromatography of a portion of the mixture indicates that no more of the desired product is being formed. Evaporate the reaction mixture, add an excess of dilute hydrochloric acid to the resultant residue, stir for 30 min and collect the insolubles. Add an excess of dilute sodiumcarbonate, stir for 30 min, collect the solids, wash with water, and dry at 60° C to obtain 9α,11β-dichloro-16α-methyl-1,4-pregnadiene-17α,21-diol-3,20-dione 17-(2'-furoate) 21-acetate.

Purify the crude product by recrystallizing from methylenechloride (75 ml)/ether (300 ml). Further purify by clarifying a methylenechloride solution of this recrystallized product by gravity filtration through charcoal (Darco® G-60), then again recrystallizing the product by adding ether (200 ml) to the methylenechloride solution adjusted to 45 ml after Darco® treatment and concentrating to 200 ml. Add an additional 50 ml

of ether, filter off the crystals, and dry at 45° C under vacuum to obtain the purified product $[\alpha]_D^{26}$+65.7° (dioxan); λ max. 245 nm (ε 23,060).

*Analysis*

| | | |
|---|---|---|
| Calculated: | C 61.8 | H 5.72 (%) |
| Found: | C 61.84 | H 5.57 (%) |

B) *9α,11β-dichloro-16β-methyl-1,4-pregnadiene-17α,21-diol-3,20-dione 17-(2'-furoate) 21-acetate*

Treat 9α,11β-dichloro-16β-methyl-1,4-pregnadiene-17α,21-diol-3,20-dione 21-acetate in a manner similar to that described in Example 1A, first paragraph, to obtain the title compound.

Purify the crude product by preparative thin-layer chromatography on silica gel, using chloroform/ethylacetate (19:1) as the developing solvent. Visualize the desired band by ultraviolet light, remove the band, and elute with ethylacetate. Evaporate the solvent and recrystallize from methylenechloride/ether to obtain purified 9α,11β-dichloro-16β-methyl-1,4-pregnadiene-17α,21-diol-3,20-dione 17-(2'-furoate) 21-acetate: λ max. 245 nm (ε 23,340); 252 nm (ε 22,990); mass spectrum (no parent ion): 491, 490, 489, 379, 351, 349, 279, 277, 95, 43.

C) *9α,11β-dichloro-16α-methyl-1,4-pregnadiene-17α,21-diol-3,20-dione 17-heterocyclic carboxylate 21-acetates*

Similarly, by substituting 3-furoylchloride, 2-thenoylchloride, and 5-bromo-2-furoylchloride for 2-furoylchloride in Example 1A, first paragraph, there are obtained (*a*) 9α,11β-dichloro-16β-methyl-1,4-pregnadiene-17α,21-diol-3,20-dione 17-(3'-furoate) 21-acetate: λ max. 237 nm (ε 17,600); mass spectrum (no parent 563-ion): (491, 489), 279, 121, 95, 43; (*b*) 9α,11β-dichloro-16α-methyl-1,4-pregnadiene-17α,21-diol-3,20-dione 17-(2'-thenoate) 21-acetate: λ max. 241 nm (ε 22,900), inflexion 270 nm; mass spectrum (no parent ion): 507, 506, 505, 380, 349, 279, 111, 43; and (*c*) 9α,11β-dichloro-16α-methyl-1,4-pregnadiene-17α,21-diol-3,20-dione 17-[2'-(5'-bromofuroate)] 21-acetate: mass spectrum (no parent ion): 569, 567, 469, 467, 452, 451, 450, 424, 351, 350, 349, 173, 43.

Each product had been purified by the preparative thin-layer chromatographic method described in Example 1B, second paragraph, followed by recrystallization of the resultant residues from ethylacetate/hexane, methylenechloride/hexane, and ethylacetate/hexane, respectively.

D) *9α,11β-dichloro-16α-methyl-1,4-pregnadiene-17α, 21-diol-3,20-dione 17-(2'-furoate) 21-propionate*

Treat 9α,11β-dichloro-16α-methyl-1,4-pregnadiene-17α,21-diol-3,20-dione 21-propionate with 2-furoylchloride in the manner of Example 1A, first paragraph, to obtain the title compound.

Purify the resultant crude product as in Example 1B, second paragraph, to obtain purified 9α,11β-dichloro-16α-methyl-1,4-pregnadiene-17α,21-diol-3,20-dione 17-(2'-furoate) 21-propionate: λ max. 244 nm (244-258 nm broad) (ε 23,000); mass spectrum (no parent ion): 491, 489, 371, 353, 351, 331, 315, 313, 279, 277, 95, 57.

*Example 2*

*9α,11β,21-trichloro-16-methyl-1,4-pregnadiene-17α-ol-3,20-dione 17-heterocyclic carboxylates*

A) *16-methyl-1,4,9(11)-pregnatriene-17α,21-diol-3,20-dione 17-heterocyclic carboxylate 21-acetates*

1) Dissolve 16α-methyl-1,4,9(11)-pregnatriene-17α,21-diol-3,20-dione 21-acetate (9.96 g, 25 mmol), 2-furoylchloride (4.95 ml, 50 mmol), and 4-dimethylaminopyridine (30.35 g, 250.0 mmol) in methylenechloride (150 ml) and stir at room temperature until thin-layer chromatography of an aliquot of the reaction mixture indicates that no more product is being formed. Evaporate the mixture and treat the residue with dilute hydrochloric acid and then with sodiumcarbonate as in Example 1A. Dissolve the collected insolubles in acetone (300 ml) and add to a saturated sodiumchloride solution to precipitate 16α-methyl-1,4,9(11)-pregnatriene-17α,21-diol-3,20-dione 17-(2'-furoate) 21-acetate.

Purify by chromatography on silica gel, eluting with chloroform/ethyl acetate (9:1). Combine like fractions as determined by thin-layer chromatography and evaporate to obtain the purified product.

2) Treat 16β-methyl-1,4,9(11)-pregnatriene-17α,21-diol-3,20-dione 21-acetate in the manner of Example 2A(1) to obtain 16β-methyl-1,4,9(11)-pregnatriene-17α, 21-diol-3,20-dione 17-(2'-furoate) 21-acetate.

3) Substitute 3-furoylchloride and 2-thenoylchloride for 2-furoylchloride in Example 2A(1) to obtain 16α-methyl-1,4,9(11)-pregnatriene-17α,21-diol-3,20-dione 21-acetate in the manner of Example 2A(1) to obtain 16β-methyl-1,4,9(11)-pregnatriene-17α, 21-diol-3,20-dione 17-(3'-furoate) 21-acetate and 16α-methyl-1,4,9(11)-pregnatriene-17α,21-diol-3,20-dione 17-(2'-thenoate) 21-acetate, respectively.

B) *16-methyl-1,4,9(11)-pregnatriene-17α,21-diol-3,20-dione 17-heterocyclic carboxylates*

1) To a suspension of the compound prepared in Example 2A(1) (4.46 g) in methanol (125 ml) at room temperature, add 70% perchloric acid (4.9 ml), dropwise. Let stand overnight. Filter off any insolubles; add the filtrate to a saturated sodiumchlorid solution, collect the solids and dry them at 45°C to obtain 16α-methyl-1,4,9(11)-pregnatriene-17α,21-diol-3,20-dione 17-(2'-furoate).

2) Hydrolyze each of the 17-heterocyclic carboxylate 21-acetates of Example 2A(2) and (3) in a similar manner to obtain the corresponding 17-heterocyclic carboxylate 21-hydroxy compounds.

C) *16-methyl-1,4,9(11)-pregnatriene-17α,21-diol-3,20-dione 17-heterocyclic carboxylate 21-mesylates*

1) To a solution of the compound prepared in Example 2B(1) (3 g) in pyridine (43 ml) cooled to 0-2°C, add dropwise mesylchloride (5.1 ml) and let stand for 1 h. Pour the reaction mixture into ice-water and collect and dry the resultant precipitate to obtain 16α-methyl-1,4,9(11)-pregnatriene-17α,21-diol-3,20-dione 17-(2'-furoate) 21-mesylate.

2) To a solution of the 16β-methyl compound prepared in Example 2B(2) (0.361 g) in pyridine (4 ml) cooled to 0-5°C, add dropwise mesylchloride (0.62 ml) and let stand for 1 h. Pour the reaction mixture into a saturated sodiumchloride solution, collect the resultant precipitate and dry it at 60°C to obtain 16β-methyl-1,4,9(11)-pregnatriene-17α,21-diol-3,20-dione 17-(2'-furoate) 21-mesylate.

3) Treat each of the 17-(3'-furoate) and 17-(2'-thenoate) compounds prepared in Example 2B(2) in a manner similar to that described in Example 2C(1) to obtain the corresponding 17-heterocyclic carboxylate 21-mesylates.

D) *21-chloro-16-methyl-1,4,9(11)-pregnatriene-17α-ol-3,20-dione 17-heterocyclic carboxylates*

1) Stir the product of Example 2C(1) (3.4 g) and lithiumchloride (3.4 g) in dimethylformamide (51 ml) at 80°C for 9 h. Add the reaction mixture to a saturated sodiumchloride solution, collect the resultant precipitate and dry it at 50°C to obtain 21-chloro-16α-methyl-1,4,9(11)-pregnatriene-17α-ol-3,20-dione 17-(2'-furoate).

2) Treat the product of Example 2C(2) in a similar manner to that described in Example 2D(1) to obtain 21-chloro-16β-methyl-1,4,9(11)-pregnatriene-17α-ol-3,20-dione 17-(2'-furoate). Purify this product by preparative thin-layer chromatography on silica gel using chloroform/ethylacetate (19:1) as developing solvent. Visualize the desired band with ultraviolet light, remove the band and elute with ethylacetate. Evaporate the solvent to obtain the purified product.

3) Treat each of the compounds prepared in Example 2C(3) in a manner similar to that described in Example 2D(1).

Purify the resulting crude 21-chloro-16α-methyl-1, 4, 9(11)-pregnatriene-17α-ol-3,20-dione 17-(3'-furoate) by recrystallization from methylenechloride/hexane, followed by preparative thin-layer chromatography using chloroform/ethylacetate (8:1) as the developing solvent.

Purify the crude 21-chloro-16α-methyl-1,4,9(11)-pregnatriene-17α-ol-3,20-dione 17-(2'-thenoate) as in Example 2D(2), second paragraph.

E) *9α,11β,21-trichloro-16-methyl-1,4-pregnadiene-17α-ol-3,20-dione 17-heterocyclic carboxylates*

1) To a solution of the product of Example 2D(1) (2.3 g, 4.0 mmol) and pyridine hydrochloride (1.42 g) in methylenechloride (37 ml) at −35 to −40°C, add carbon tetrachloride containing chlorine (3.26 ml; 128 mg $Cl_2$/ml) and stir for 20 min. Evaporate the solvent, add water to the resultant residue, and collect the insolubles to obtain 9α, 11β, 21-trichloro-16α-methyl-1,4-pregnadiene-17α-ol-3,20-dione 17-(2'-furoate).

Purify the product by recrystallization from methylenechloride/ether, followed by preparative thin-layer chromatography on silica gel using chloroform/ethylacetate (9:1) as the developing solvent. Visualize the desired band with ultraviolet light, remove the band and elute with ethylacetate. Evaporate the solvent to obtain the purified product: λ max. 245.5 nm (ε 24,300), shoulder 253 nm.

*Analysis*

| | | | |
|---|---|---|---|
| Calculated: | C 60.08 | H 5.41 | Cl 19.71(%) |
| Found: | C 60.37 | H 5.49 | Cl 19.59(%) |

2) In a manner similar to that described in Example 2E(1), treat each of the products of Example 2D(2), (3) and (4) with chlorine in carbon tetrachloride to obtain the corresponding 9α,11β,21-trichloro 17-heterocyclic carboxylates, i.e. 9α,11β,21-trichloro-16β-methyl-1,4-pregnadiene-17α-ol-3,20-dione 17-(2'-furoate); 9α, 11β,21-trichloro-16α-methyl-1,4-pregnadiene-17α-ol-3,20-dione 17(3'-furoate): λ max. 236 nm (ε 16,300).

*Analysis*

| | | |
|---|---|---|
| Calculated: | C 60.06 | H 5.41(%) |
| Found: | C 59.77 | H 5.29(%) |

9α,11β,21-trichloro-16α-methyl-1,4-pregnadiene-17α-ol-3,20-dione 17-(2'-thenoate): λ max. 240 nm (ε 22,390).

*Analysis*

| | | | |
|---|---|---|---|
| Calculated: | C 58.33 | H 5.26 | Cl 19.13(%) |
| Found: | C 57.90 | H 5.10 | Cl 19.34(%) |

*Example 3*

*9α-fluoro-16-methyl-1,4-pregnadiene-11β,17α,21-triol-3,20-dione 17-heterocyclic carboxylates*

A) *9α-fluoro-16-methyl-1,4-pregnadiene-17α,21-diol-3,11,20-trione 17-heterocyclic carboxylate 21-acetates*

1) To a solution of 4-dimethylaminopyridine (8.4 g, 70 mmol) in methylenechloride (42 ml), add dropwise 2-furoylchloride (1.8 ml, 18.2 mmol) with stirring. Add 9α-fluoro-16α-methyl-1,4-pregnadiene-17α,21-diol-3,11,20-trione 21-acetate (3 g, 6.9 mmol) and stir at room temperature until thin-layer chromatography of a portion of the reaction mixture indicates that no more product is being formed. Evaporate the reaction mixture and treat the resultant residue by successive trituration with dilute hydrochloric acid and with dilute sodiumcarbonate; collect the insolubles, wash with water, and dry under

vacuum at 40°C to obtain 9α-fluoro-16α-methyl-1,4-pregnadiene-17α,21-diol-3,11,20-trione 17-(2'-furoate) 21-acetate.

Purify the crude product by preparative thin-layer chromatography on silica gel: develop the plates twice in chloroform/ethylacetate (40:1), visualize the desired band by ultraviolet light, remove the band and elute with ethylacetate. Evaporate the eluate to a residue to obtain purified 9α-fluoro-16α-methyl-1,4-pregnadiene-17α,21-diol-3,11,20-trione 17-(2'-furoate) 21-acetate.

2) In the procedure of Example 3A(1), substitute for 2-furoylchloride equivalent amounts of 3-furoylchloride and 2-thenoylchloride to obtain 9α-fluoro-16α-methyl-1,4-pregnadiene-17α,21-diol-3,11,20-trione 17-(3'-furoate) 21-acetate and 9α-fluoro-16α-methyl-1,4-pregnadiene-17α,21-diol-3,11,20-trione 17-(2'-thenoate) 21-acetate, respectively.

3) Treat 9α-fluoro-16β-methyl-1,4-pregnadiene-17α,21-diol-3,11,20-trione 21-acetate in a manner similar to that described in Example 3A(1), first paragraph, but substitute dimethylformamide/methylenechloride (1:1) for methylenechloride.

Purify the crude 9α-fluoro-16β-methyl-1,4-pregnadiene-17α,21-diol-3,11,20-trione 17-(2'-furoate) 21-acetate as in Example 3A(1), second paragraph, followed by a second preparative thin-layer chromatographic purification using hexane/ethylacetate (2:1) as the developing solvent.

B) *9α-fluoro-16-methyl-1,4-pregnadiene-11β,17α,21-triol-3,20-dione 17-heterocyclic carboxylate 21-acetates*

1) To a solution of the compound prepared in Example 3A(1) (0.986 g, 1.866 mmol) in dimethylformamide (26 ml), methanol (30 ml) and water (3 ml), cooled to 0-2°C under an atmosphere of nitrogen, add solid sodiumborohydride (0.212 g, 5.56 mmol). After 20 min, add 1N hydrochloric acid (6 ml), wait 1 min, and pour the reaction mixture into a saturated sodiumchloride solution (600 ml). Collect the precipitate and dry it at 60°C.

Purify the crude product by preparative thin-layer chromatography on silica gel, using chloroform/ethylacetate (9:1) to develop the plates. Visualize the desired band by ultraviolet light, remove the band, and elute with ethylacetate. Evaporate the solvent and recrystallize the resultant residue from methylenechloride/ether to obtain 9α-fluoro-16α-methyl-1,4-pregnadiene-11β,17α,21-triol-3,20-dione 17-(2'-furoate) 21-acetate; λ max. 247 nm (ε 25,890).

*Analysis*

| | | | |
|---|---|---|---|
| Calculated: | C 65.89 | H 6.29 | F 3.59(%) |
| Found: | C 65.92 | H 6.23 | F 3.58(%) |

2) In the procedure of Example 3B(1), first paragraph, substitute the products of Example 3A(2) for the 17-(2'-furoate), substitute argon for nitrogen, and run at 0-5°C to obtain 9α-fluoro-16α-methyl-1,4-pregnadiene-11β,17α,21-triol-3,20-dione 17-(3'-furoate) 21-acetate and 9α-fluoro-16α-methyl-1,4-pregnadiene-11β, 17α, 21-triol-3,20-dione 17-(2'-thenoate) 21-acetate, respectively.

Purify the crude 17-(3'-furoate) and 17-(2'-thenoate) by preparative thin-layer chromatography as in Example 3B(1), second paragraph, substituting chloroform/ethylacetate at ratios of (19:1) and (13:1) for the developing solvents, respectively to obtain the purified 17-(3'-furoate): λ max. 237 nm (ε 16,700); $[\alpha]_D^{26}+9.0°$ (dioxan); mass spectrum (no parent ion): 508, 456, 455, 397, 396, 395, 279, 278, 215, 187, 112, 95, 43; and the purified 17-(2'-thenoate) λ max. 242 nm (ε 22,310), 270 nm (inflexion) (ε 11,373); mass spectrum (no parent ion): 524, 471, 315, 295, 277, 128, 111, 73, 43.

3) To a solution of the compound prepared in Example 3A(3) (26 mg, 0.0494 mmol) in methanol (2.5 ml) and water (0.3 ml), cooled to 0-5°C under a nitrogen atmosphere, add sodiumborohydride (7 mg, 0.148 mmol). After 20 min, add to dilute hydrochloric acid and extract with ethylacetate to obtain 9α-fluoro-16β-methyl-1,4-pregnadiene-11β,17α,21-triol-3,20-dione 17-(2'-furoate) 21-acetate.

Purify the crude product using preparative thin-layer chromatography as in Example 3B(1), second paragraph, substituting chloroform/ethylacetate (19:1) for the developing solvent to obtain the purified product: λ max. 248 nm (ε 25,500); mass spectrum (no parent ion): 508, 455, 396, 315, 295, 277, 112, 95, 43.

C) *9α-fluoro-16-methyl-1,4-pregnadiene-11β,17α,21-triol-3,20-dione 17-heterocyclic carboxylates*

1) To a suspension of the compound prepared in Example 3B(1) (334 mg, 0.634 mmol) in methanol (9 ml) under an atmosphere of nitrogen, add with stirring 70% perchloric acid (0.34 ml). After 18 h separate the insolubles and add the clear reaction mixture to a saturated aqueous sodiumchloride solution (150 ml); collect the precipitate and dry it at 60°C to obtain 9α-fluoro-16α-methyl-1,4-pregnadiene-11β,17α,21-triol-3,20-dione 17-(2'-furoate).

2) Treat the compound prepared in Example 3B(3) in a manner similar to that described in Example 3C(1) to obtain 9α-fluoro-16β-methyl-1,4-pregnadiene-11β,17α,21-triol-3,20-dione 17-(2'-furoate).

Purify the crude product by recrystallization from ethylacetate/hexane, then by preparative thin-layer chromatography in the usual manner, using chloroform/ethylacetate (4:1) as developing solvent.

3) In the procedure of Example 3C(1) substitute for the 17-(2'-furoate) equivalent amounts of 17-(3'-furoate) and 17-(2'-thenoate) to obtain the corresponding 9α-fluoro-16α-methyl-1,4-pregnadiene-11β,17α,21-triol-3,20-dione 17-(3'-furoate) and 9α-fluoro-16α-methyl-1,4-pregnadiene-11β,17α,21-triol-3,20-dione 17-(2'-thenoate).

D) *9α-fluoro-16α-methyl-1,4-pregnadiene-11β,17α,21-triol-3,20-dione 17-(2'-furoate) 21-propionate*

1) To a solution of the compound of Example 3C(1) (0.1 g, 0.021 mmol) in pyridine (3 ml) cooled to 0-2°C, add propionylchloride (0.3 ml, 0.035 mmol). After 17 h, add to dilute hydrochloric acid and extract with ethylacetate. Evaporate the ethylacetate extract to give a residue comprising 9α-fluoro-16α-methyl-1,4-pregnadiene-11β,17α,21-triol-3,20-dione 17-(2'-furoate) 21-propionate.

Purify the crude product by preparative thin-layer chromatography using chloroform/ethylacetate (8:1) as developing solvent, followed by recrystallization from methylenechloride/hexane: λ max. 246 nm (ε 26,300); mass spectrum (no parent ion): 522, 457, 456, 455, 427, 374, 315, 295, 277, 95, 57.

2) In the procedure of 3D(1), treat the products of Examples 3C(2) and (3) with propionylchloride to obtain the corresponding 17-heterocyclic carboxylate 21-propionates.

*Example 4*

*9α-fluoro-21-chloro-16-methyl-1,4-pregnadiene-11β,17α-diol-3,20-dione 17-heterocyclic carboxylates*

A) *9α-fluoro-16α-methyl-1,4-pregnadiene-11β,17α,21-triol-3,20-dione 17-heterocyclic carboxylate 21-mesylates*

1) Dissolve the compound of Example 3C(1) (269 mg, 0.553 mmol) in a mixture of mesylchloride (0.43 ml, 5.53 mmol) and pyridine (2.75 ml) maintained at 0-2°C. After 1 h, add the reaction mixture to a saturated sodiumchloride solution. Collect the insoluble material and dry it at 40°C to obtain 9α-fluoro-16α-methyl-1,4-pregnadiene-11β,17α,21-triol-3,20-dione 17-(2'-furoate) 21-mesylate.

2) In a similar manner to Example 4A(1), treat the compounds prepared in Examples 3C(2) and (3) to obtain the corresponding 17-heterocyclic carboxylate 21-mesylates.

B) *9α-fluoro-21-chloro-16α-methyl-1,4-pregnadiene-11β,17α-diol-3,20-dione 17-heterocyclic carboxylates*

1) Dissolve the compound of Example 4A(1) (279 mg, 0.494 mmol) and lithiumchloride (350 mg) in dimethylformamide (4 ml) and maintain the temperature at 80°C for 21 h. Add the reaction mixture to a satured sodiumchloride solution, collect the insolubles and dry at 50°C to obtain 9α-fluoro-21-chloro-16α-methyl-1,4-pregnadiene-11β,17α-diol-3,20-dione 17-(2'-furoate).

Purify the product by preparative thin-layer chromatography using chloroform/ethylacetate (8:1) as developing solvent. Recrystallize the resultant chromatographic extract from methylenechloride/hexane to obtain the purified title compound: λ max. 247 nm (ε 25,210).

*Analysis*

| | | |
|---|---|---|
| Calculated: | C 64.22 | H 5.99(%) |
| Found: | C 64.13 | H 5.66(%) |

2) Treat the compounds prepared in Example 4A(2) in the manner of Example 4B(1) to obtain the corresponding 21-chloro 17-heterocyclic carboxylate, i.e. 9α-fluoro-21-chloro-16β-methyl-1,4-pregnadiene-11β,17α-diol-3,20-dione 17-(2'-furoate): λ max. 248 nm (ε 24,800); mass spectrum (no parent ion): 486, 485, 484, 374, 373, 372, 317, 316, 315, 297, 296, 295, 95, 43; 9α-fluoro-21-chloro-16α-methyl-1,4-pregnadiene-11β,17α-diol-3,20-dione 17-(3'-furoate): λ max. 238 nm (ε 18,400); mass spectrum (no parent ion): 484, 372, 295, 277, 95; and 9α-fluoro-21-chloro-16α-methyl-1,4-pregnadiene-11β,17α-diol-3,20-dione 17-(2'-thenoate): λ max. 243 nm (ε 24,460), inflexion at 260 and 272 nm.

*Analysis*

| | | | | |
|---|---|---|---|---|
| Calculated: | C 62.24 | H 5.80 | S 6.15 | F 3.65(%) |
| Found: | C 62.07 | H 5.73 | S 6.59 | F 3.53(%) |

*Example 5*

*1,4-pregnadiene-11β,17α,21-triol-3,20-dione 17-(2'-furoate) 21-acetate and its 9α-fluoro derivative*

A) *1,4-pregnadiene-17α,21-diol-3,11,20-trione 17-(2'-furoate) 21-acetate*

Treat 1,4-pregnadiene-17α,21-diol-3,11,20-trione 21-acetate in a manner similar to that described in Example 1A, first paragraph, to obtain the title compound.

Purify the product by successive preparative thin-layer chromatography procedures, the first using chloroform/ethylacetate (9:1) as developing solvent, the second using hexane/ethylacetate (2:1).

B) *1,4-pregnadiene-11β,17α,21-triol-3,20-dione 17-(2'-furoate) 21-acetate*

To a solution of the compound of Example 5A (148 mg) in methanol (15 ml) dimethylformamide (10 ml), and water (1.5 ml), cooled to 0-2°C under a nitrogen atmosphere, add sodiumborohydride (34.1 mg). After 20 min, add to dilute hydrochloric acid (250 ml) and collect the insolubles. Extract the aqueous solution with ethylacetate, evaporate the organic phase and combine the residue and insolubles to give 1,4-pregnadiene-11β,17α,21-triol-3,20-dione 17-(2'-furoate) 21-acetate.

Purify the crude product by preparative thin-layer chromatography in the usual manner using chloroform/ethylacetate (4:1) as the developing solvent, followed by recrystallizing from methylenechloride/hexane to obtain the purified product: λ max. 249 nm (ε 26,510); mass spectrum (no parent ion): 496, 384, 283, 265, 250, 237, 223, 95, 43.

C) *9α-fluoro-1,4-pregnadiene-11β,17α,21-triol-3,20-dione 17-(2'-furoate) 21-acetate*

Treat 9α-fluoro-1,4-pregnadiene-17α,21-triol-

3,11,20-trione 21-acetate with 2-furoylchloride and purify the product as described in Example 5A. Reduce the 17-(2'-furoate) thus prepared with sodiumborohydride and purify as described in Example 5B to produce the title compound.

*Example 6*

*16-methyl-1,4-pregnadiene-11β,17α,21-triol-3,20-dione 17-(2'-furoate) 21-acetates*

Treat each of 16α-methyl-1,4-pregnadiene-17α,21-diol-3,11,20-trione 21-acetate and 16β-methyl-1,4-pregnadiene-17α,21-diol-3,11,20-trione 21-acetate in a manner similar to those of Examples 5A and 5B to obtain the corresponding 16α-methyl-1,4-pregnadiene-11β,17α,21-triol-3,20-dione 17-(2'-furoate) 21-acetate and 16β-methyl-1,4-pregnadiene-11β, 17α, 21-triol-3,20-dione 17-(2'-furoate) 21-acetate.

*Example 7*

*9α,11β-dichloro-16α-methyl-1,4-pregnadiene-17α,21-diol-3,20-dione 17-(5'-methyl-2'-thenoate) 21-acetate*

Dissolve 4-dimethylaminopyridine (3 g), 9α,11β-dichloro-16α-methyl-1,4-pregnadiene-17α,21-diol-3,20-dione 21-acetate (1 g), and 5-methyl-2-thenoylchloride (0.6 ml) in methylenechloride (10 ml) and stir at room temperature until thin-layer chromatography of a portion of the mixture indicates no more of the desired product is being formed. Dilute the resultant mixture with methylenechloride (200 ml), add dilute hydrochloric acid and stir for 45 min. Separate the methylenechloride phase, wash it with dilute sodiumcarbonate and then with water, and evaporate the organic phase.

Purify the crude product by preparative thin-layer chromatography on silica gel using chloroform/ethylacetate (40:1) as developing solvent. Visualize the desired band by ultraviolet light, remove the band, and elute with ethylacetate. Repeat the preparative thin-layer chromatography, developing with chloroform/ethylacetate (15:1). Elute with ethylacetate, evaporate the solvent, and recrystallize the resultant residue from methylenechloride/ether to obtain pure 9α,11β-dichloro-16α-methyl-1,4-pregnadiene-17α,21-diol-3,20-dione 17-(5'-methyl-2'-thenoate) 21-acetate: λ max. 241 nm (ε 20,570), 277 nm (ε 14,100); mass spectrum (no parent ion): 519, 349, 279, 126, 125, 43.

*Example 8*

*9α-fluoro-16α-methyl-1,4-pregnadiene-11β,17α,21-triol-3,20-dione 17-(5'-methyl-2'-thenoate) 21-acetate*

Treat 9α-fluoro-16α-methyl-1,4-pregnadiene-11β,17α,21-triol-3,20-dione 21-acetate in a manner similar to that described in Example 7, first paragraph, but with the addition of 2.5 ml dimethylformamide, to obtain the title compound.

Purify the crude product by preparative thin-layer chromatography as usual, developing with chloroform/ethylacetate (50:1). Purify further by preparative thin-layer chromatography using chloroform/ethylacetate (10:1) as developing solvent and recrystallize the resultant residue from methylenechloride/hexane to give a purified product: λ max. 244 nm (ε 21,770), 277 nm (ε 13,450); mass spectrum (no parent ion): 487, 485, 374, 343, 316, 315, 296, 125, 43.

*Example 9*

*9α,11β-dichloro-16α-methyl-1,4-pregnadiene-17α,21-diol-3,20-dione 17-(N-methyl-2'-pyrrolylcarboxylate) 21-acetate*

Dissolve 9α,11β-dichloro-16α-methyl-1,4-pregnadiene-17α,21-diol-3,20-dione 21-acetate (3 g) in methylenechloride (30 ml) and add 4-dimethylaminopyridine (8.4 g) and N-methylpyrrole-2-carbonylchloride (2 ml). Stir at room temperature until thin-layer chromatography of a portion of the mixture indicates that no more of the desired product is being formed. Evaporate the reaction mixture, add dilute sodiumcarbonate, and stir 1 h. Extract the solution three times with 200 ml methylenechloride, combine the organic phases, wash them with water, and evaporate them to a residue to obtain the title compound.

Purify the crude compound by silica gel chromatography, eluting with ethylacetate. Combine like fractions as determined by thin-layer chromatography, and evaporate the solvent to obtain the title compound. Purify further by preparative thin-layer chromatography, using chloroform/ethylacetate (20:1) as developing solvent. Extract the sample band as usual with ethylacetate, evaporate, and recrystallize the resultant residue from ether to obtain the pure title compound; mass spectrum (no parent ion): 502, 279, 277, 271, 142, 108, 43.

*Example 10*

*6α-fluoro-16α-methyl-1,4-pregnadiene-17α-ol-3,20-dione 17-aromatic heterocyclic carboxylates*

A) *6α-fluoro-16α-methyl-1,4-pregnadiene-11β,17α,21-triol-3,20-dione 17-(2'-furoate) 21-acetate*

1) *6α-fluoro-16α-methyl-1,4-pregnadiene-17α,21-diol-3,11,20-trione 17-(2'-furoate) 21-acetate*

Dissolve 4-dimethylaminopyridine (9 g) and 2-furoylchloride (2.1 ml) in methylenechloride (40 ml), add 6α-fluoro-16α-methyl-1,4-pregnadiene-17α,21-diol-3,11,20-trione 21-acetate (2.9 g) in methylenechloride (20 ml) and stir at room temperature for 96 h. Dilute the reaction mixture with methylenechloride (300 ml), then wash with dilute hydrochloric acid. Separate the organic phase, dry it over anhydrous sodiumsulfate, and evaporate it to a residue.

Dissolve the resultant residue in methylenechloride (100 ml) and filter the solution through neutral aluminium oxide to obtain the purified title compound. Purify further by preparative thin-layer chromatography using ethylacetate/hexane (1:1)

to develop the plates, extract the band as usual with ethylacetate, and evaporate the extract to a residue to obtain the purified product.

2) *6α-fluoro-16α-methyl-1,4-pregnadiene-11β,17α,21-triol-3,20-dione 17-(2'-furoate) 21-acetate*

Dissolve the product of Example 10A(1) (0.360 g) in dimethylformamide (10 ml) and methanol (10 ml). Cool the solution to 0°C, and under a nitrogen atmosphere add sodiumborohydride (0.073 g). Stir for 30 min at 0°C. Add dilute hydrochloric acid (18 ml) to the reaction mixture and pour the resultant solution into ice-water saturated with sodiumchloride. Collect the resultant solids and purify by preparative thin-layer chromatography using chloroform/ethylacetate (2:1) as developing solvent. Extract the product as usual with ethylacetate, evaporate to a residue, and recrystallize it from ethylacetate/hexane (3:1) to obtain the purified title compound: λ max. 247 nm (ε 27,800); mass spectrum: 528, 455, 315, 112, 95, 43.

B) *6α-fluoro-9α,11β-dichloro-16α-methyl-1,4-pregnadiene-17α,21-diol-3,20-dione 17-(2'-furoate) 21-acetate*

1) *6α-fluoro-16α-methyl-1,4,9(11)-pregnatriene-17α,21-diol-3,20-dione 21-acetate*

Under a nitrogen atmosphere cool to 0°C a solution of 6α-fluoro-16α-methyl-1,4-pregnadiene-11β,17α,21-triol-3,20-dione 21-acetate (12.5 g) in dimethylformamide (25 ml) and collidine (25 ml). Add slowly methanesulfonylchloride/$SO_2$ solution (1.41 g $CH_3SO_2Cl$/ml), stir for 45 min at 0°C, and then stir for 30 min at room temperature. Pour the resultant reaction mixture into ice-water (1.1 l), collect the insolubles and wash these with water to obtain the crude title compound. Dissolve the residue in methylenechloride, filter through silica gel, and evaporate the solvent to obtain the purified product.

2) *6α-fluoro-16α-methyl-1,4,9(11)-pregnatriene-17α,21-diol-3,20-dione 17-(2'-furoate) 21-acetate*

Treat the product of Example 10B(1) in a manner similar to that described in Example 2A(1), first paragraph, to obtain the title compound. Purify the crude product as usual by preparative thin-layer chromatography, developing with hexane/ethylacetate (2:1).

3) *6α-fluoro-9α,11β-dichloro-16α-methyl-1,4-pregnadiene-17α,21-diol-3,20-dione 17-(2'-furoate) 21-acetate*

Treat the product of Example 10B(2) in a manner similar to that described in Example 2E(1), substituting hexane/ethylacetate (2:1) as solvent, and purify further by recrystallizing from methylenechloride/hexane, to obtain the title compound: λ max. 243 nm (ε 23,300); mass spectrum (no parent ion): 509, 507, 398, 397, 395, 297, 289, 287, 269, 267, 229, 95, 43.

C) *6α-fluoro-9α,11β,21-trichloro-16α-methyl-1,4-pregnadiene-17α-ol-3,20-dione 17-(2'-furoate)*

1) *6α-fluoro-16α-methyl-1,4,9(11)-pregnatriene-17α,21-diol-3,20-dione 17-(2'-furoate)*

Treat the product of Example 10B(2) in a manner similar to that described in Example 2B(1) to obtain the title compound. Purify by preparative thin-layer chromatography as usual, using chloroform/ethylacetate (9:1) to develop the plates, and hexane/ethylacetate (2:1) to recrystallize the purified title compound.

2) *6α-fluoro-16α-methyl-1,4,9(11)-pregnatriene-17α,21-diol-3,20-dione 17-(2'-furoate) 21-mesylate*

Dissolve the product of Example 10C(1) (4.4 g) in pyridine (30 ml), add mesylchloride (5 ml) cooled to 0-2°C, and stir 1 h at room temperature under a nitrogen atmosphere. Pour the reaction mixture into dilute hydrochloric acid (300 ml) and collect and dry the insolubles to obtain the title compound.

3) *6α-fluoro-21-chloro-16α-methyl-1,4,9(11)-pregnatriene-17α-ol-3,20-dione 17-(2'-furoate)*

Treat the compound prepared in Example 10C (2) in a manner similar to that described in Example 2D(1) to obtain the title compound.

4) *6α-fluoro-9α,11β,21-trichloro-16α-methyl-1,4-pregnadiene-17α-ol-3,20-dione 17-(2'-furoate)*

Treat the compound prepared in Example 10C (3) in a manner similar to that described in Example 2E(1)first paragraph, but, rather than evaporating the organic solvent, wash the reaction mixture with water, dry the organic phase over anhydrous sodiumsolvate, and evaporate to a residue.

Purify the resultant residue as usual by preparative thin-layer chromatography, using chloroform/ethylacetate (19:1) to develop the plates and ethylacetate to extract the product. Recrystallize from methylenechloride/hexane to obtain the purified title compound: λ max. 243 nm (ε 23,000), inflexion 255 nm; mass spectrum: 556, 521, 510, 509, 507, 481, 479, 317, 95.

D) *6α-fluoro-9α,21-dichloro-16α-methyl-1,4-pregnadiene-11β,17α-diol-3,20-dione 17-(2'-furoate)*

Dissolve the compound prepared in Example 10C(3) (0.974 g) in tetrahydrofuran (25 ml) and cool to 15°C. Add perchloric acid (0.3 ml of 70% perchloric acid in 0.7 ml water) and 1,3-dichloro-5,5-dimethylhydantoin (0.237 g), and stir under a nitrogen atmosphere at room temperature for 2 h. Add the reaction mixture to a solution of sodiumbisulfite in water (2 g in 250 ml) and collect the solids to obtain the title compound. Purify the crude product by chromatography on silica gel G-60, combine the desired fractions as determined by thin-layer chromatography and evaporate to

give the purified product: λ max. 245 nm (ε 23,000); mass spectrum (no parent ion): 489, 349, 313, 293, 112, 95, 77, 35.

*Example 11*

*9α,11β,dichloro-16α-methyl-1,4-pregnadiene-17α,21-diol-3,20-dione 17-(2'-thenoate)*

A) *9α,11β-dichloro-16α-methyl-1,4-pregnadiene-17α,21-diol-3,20-dione 17α,21-(2-methylorthothenoate)*

Dissolve 9α,11β-dichloro-16α-methyl-1,4-pregnadiene-17α, 21-diol-3,20-dione (2 g) in a mixture of dioxan (20 ml) and benzene (60 ml) and reflux using a Dean-Stark take-off collector. After distilling off 20 ml solvent, add 2-trimethylorthothenoate dissolved in benzene (1.82 g in 10 ml) and pyridinium p-toluenesulfonate (0.072 g). Reflux for 10 min with distillation and concurrent replacement of benzene. Repeat the addition of orthoester and pyridiniumtosylate and the distillation step four more times.

Cool the reaction mixture to room temperature, add 3 drops of pyridine and evaporate *in vacuo* to a residue comprising the title compound.

B) *9α,11β-dichloro-16α-methyl-1,4-pregnadiene-17α,21-diol-3,20-dione 17-(2'-thenoate)*

Dissolve the residue prepared in Example 11A in acetic acid (35 ml of a 90% solution in water) and stir at room temperature for 24 h. Add the reaction mixture to water (300 ml) and extract with ethylacetate to obtain the title compound. Purify the crude material by recrystallization from methylenechloride/hexane and by preparative thin-layer chromatography as usual, using chloroform/ethylacetate (9:1) as developing solvent to obtain the pure title compound: λ max. 241 nm (ε 22,310), inflexions at 251, 258 and 263 nm; mass spectrum (no parent ion): 507, 505, 317, 315, 279, 111, 91, 83.

*Example 12*

*9α-fluoro-16α-methyl-1,4-pregnadiene-11β,17α,21-triol 3,20-dione 17-(2'-furoate) 21-methoxyacetate*

Cool a solution of 0.115 g of 9α-fluoro-16α-methyl-1,4-pregnadiene-11β,17α,21-triol-3,20-dione 17-(2'-furoate), prepared in Example 3C (1), in 1 ml of pyridine to 5° C, and then add 0.05 ml of methoxyacetylchloride. After 5 min, allow the temperature to rise to room temperature and then stand for 75 min. Add the reaction mixture to aqueous hydrochloric acid and collect the insoluble material (100 mg). Purify by preparative thin-layer chromatography using a mixture of chloroform/ethylacetate (2.5:1) to develop the plates, extract the band with ethylacetate and evaporate the extract to give the title compound (91 mg: yield 68% of the theory). Further recrystallization may be effected using an ethylacetate/hexane mixture. Title compound: mass spectrum (no parent ion): 540, 539, 538, 426, 315, 295, 95, 45.

The corresponding 21-methylthioacetate analog of the title compound may similarly be prepared using methylthioacetylchloride in place of methoxyacetylchloride.

*Example 13*

*9α,11β-dichloro-17α,21-dihydroxy-16α-methyl-1,4-pregnadiene-3,20-dione 17-(2'-furoate) 21-methoxyacetate*

To 5 ml of pyridine at 0-2° C add with stirring 0.15 ml methoxyacetylchloride. To the resulting suspension add 522 mg of 9α,11β-dichloro-17α,21-dihydroxy-16α-methyl-1,4-pregnadiene-3,20-dione 17-(2'-furoate). After 5 min, allow the temperature of the reaction mixture to rise to room temperature and maintain, under stirring, for 3 h. Add the product mixture to distilled water, saturate with sodiumchloride and then filter off the white precipitate, wash and then dry under vacuum at 50° C to give 594 mg of product (theory yield). Recrystallize the crude product twice from a methylenechloride/ether mixture at reflux temperature.

Further purify the product by preparative thin-layer chromatography on 1,000 μm silica gel plates using chloroform/ethylacetate (9:1) as developing solvent. Elute the desired band with ethylacetate, filter the eluant, remove the solvent by evaporation at room temperature and then dry the residue under vacuum at 50° C to give 465 mg of product (yield 78% of the theory). Recrystallize the product from a methylenechloride/hexane mixture at reflux temperature to give white needles of the pure title product (384 mg; yield 65% of the theory): λ max. 245 nm (ε 23,280); mass spectrum: 522, 491, 489, 410, 379, 377, 351, 349, 315, 313, 279, 277, 121, 112, 95.

*Example 14*

*9α-21-dichloro-11β,17α-dihydroxy-16α-methyl-1,4-pregnadiene-3,20-dione 17-(2'-furoate)*

Prepare under nitrogen a solution of 1.80 g of 21-chloro-17α-hydroxy-16α-methyl-1,4,9(11)-pregnatriene-3,20-dione 17-(2'-furoate), obtained in a manner similar to that described in Example 10C(3), in 39 ml of dry tetrahydrofuran.

Maintain under nitrogen and cool on an ice-bath. Add, with stirring, a solution of 1.15 ml of 70% perchloric acid in 2.53 ml of distilled water, and immediately thereafter 604 mg of 1,3 dichloro-5,5-dimethylhydantoin. Stir the reaction mixture for 20 min and then raise the temperature to ambient temperature. Monitor the consumption of starting material by thin-layer chromatography of aliquots using chloroform/ethylacetate (9:1) and hexane/ethylacetate (1:1). We found that a sample, taken 2 h after the hydantoin reactant addition, indicated total consumption of starting material. At 2½ h after hydantoin addition, the reaction mixture was poured into 500 ml of distilled water containing 7 g of sodiumbisulphite. Sodiumchloride was added until the solution was saturated. The precipitated solid was filtered off,

washed copiously and then dried at 50° C under vacuum.

The resulting crude product was purified by preparative chromatography on 1,000 µm silica gel plates using chloroform/ethylacetate (19:1). The desired band was eluted with ethylacetate, the eluate filtered and then evaporated at room temperature to give 1.3 g of product (yield 65% of the theory). The product was recrystallized by dissolving in refluxing methylenechloride, filtering and then replacing the methylenechloride at reflux with methanol and then the methanol with distilled water. Self-seeding occurred. The suspension was cooled to room temperature, filtered and then dried under vacuum at 50° C to give the pure title product: λ max. 247 nm (ε 24,940); mass spectrum (no parent ion): 486, 484, 374, 372, 331, 313, 295, 277, 121, 95.

*Example 15*

*9α-chloro-21-fluoro-11β,17α-dihydroxy-16α-methyl-1,4-pregnadiene-3,20-dione 17-(2'-furoate)*

A) *21-fluoro-17α-hydroxy-16α-methyl-1,4,9-(11)-pregnatriene-3,20-dione 17-(2'-furoate)*

Stir at room temperature a mixture of 1.411 g of 21-fluoro-17α-hydroxy-16α-methyl-1,4,9(11)-pregnatriene-3,20-dione [made by the procedure of Herz *et al.*, JACS, *78*, 4812 (1956)], 1.623 g of furoic anhydride and 1.923 g of 4-dimethylaminopyridine in 16 ml of methylenechloride for 5 d until thin-layer chromatography indicates 80-85% reaction. Air-evaporate the methylenechloride, triturate the residue with water and then collect the solid product by filtration. Dry the product at 50° C under vacuum (yield 1.79 g). Purify the crude product by preparative thin-layer chromatography on 1,000 µm silica gel plates using a chloroform/ethylacetate mixture (first 9:1 and then 19:1 ratio) as developing solvent. Elute the desired band with ethylacetate, filter the eluate and evaporate at room temperature to give a residue of 1.35 g (yield 75.8% of the theory). Recrystallize the product twice from methylenechloride to yield white needles of 21-fluoro-17α-hydroxy-16α-methyl-1,4,9(11) -pregnatriene- 3,20-dione 17-(2'-furoate) (764 mg; yield 43% of the theory): λ max. 246.5 nm (ε 25,430); mass spectrum: 452, 437, 340, 325, 307, 279, 224, 171, 95.

B) *9α-chloro-21-fluoro-11β,17α-dihydroxy-16α-methyl-1,4-pregnadiene-3,20-dione 17-(2'-fuorate)*

To a cooled solution (0-2° C) maintained under nitrogen of 538.5 mg of the product obtained by step A in 12 ml of tetrahydrofuran, add with stirring a solution of 0.36 ml of 70% perchloric acid in 0.8 ml of distilled water and immediately thereafter 187.5 mg of 1,3-dichloro-5,5'-dimethylhydantoin. After 5 min, remove the reaction mixture from the ice-bath, discontinue the nitrogen flow and stir the reaction at room temperature for 150 min until consumption of the starting material is substantially complete as indicated by thin-layer chromatography of aliquots using hexane/ethylacetate (2:1). Pour the product mixture into 700 ml of distilled water containing 2 g of sodiumbisulphite. Add sodiumchloride until the solution is saturated.

Filter off the precipitate, wash copiously with water and then dry at 60° C under vacuum to give 597 mg of crude product (yield 96% of the theory).

Purify the crude product by preparative thin-layer chromatography on 1,000 µm silica gel plates using chloroform/ethylacetate (9:1). Elute the desired band with ethylacetate, filter the eluate, evaporate off the solvent and then triturate the residue with ether, discarding the supernatant liquor. Dry the product at room temperature (yield 440 mg). Recrystallize from a methylenechloride/hexane mixture at reflux temperature to give 375 mg of the pure title compound: λ max. 246 nm (methanol) (ε 25,730); mass spectrum: 505, 504, 469, 468, 356, 331, 313, 295, 277, 121, 95.

*Example 16*

*9α-chloro-11β,17α-dihydroxy-16α-methyl-21-thiol-1,4-pregnadiene-3,20-dione 17-(2'-furoate) 21-pivalate*

A) *9β-11β-epoxy-17α-hydroxy-16α-methyl-21-thiol-1,4-pregnadiene-3,20-dione 17-(2'-furoate) 21-pivalate*

Prepare a mixture of 236.3 mg of 9β,11β-epoxy-17α-hydroxy-16α-methyl-21-thio-1,4-pregnadiene-3,20-dione 21-pivalate (prepared according to the procedure of British patent specification No. 2037290A), 206 mg of furoic anhydride and 244.4 mg of 4-dimethylaminopyridine in 2 ml of methylenechloride. Stir the mixture in a stoppered 2-dram vial for 160 min at which time the reaction should be substantially complete as indicated by thin-layer chromatography of a sample on silica gel plates using as eluant chloroform/ethylacetate (19:1) and then hexane/ethylacetate (2:1). Evaporate the product mixture at room temperature and triturate the residue with distilled water. Filter off the insolubles and dry at 60° C under vacuum.

Purify the crude product by preparative thin-layer chromatography on 1,000 µm silica gel plates using as eluant chloroform/ethylacetate. Extract the desired band with ethylacetate and evaporate the resulting extracts at room temperature. Recrystallize from a methylenechloride/diethylether/hexane mixture to give 229 mg (yield 81% of the thoery) of 9β,11β-epoxy-17α-hydroxy-16α-methyl-21-thiol-1,4-pregnadiene-3,20-dione 17-(2'-furoate) 21-pivalate: λ max. 250 nm (methanol) (ε 30,320); mass spectrum: 556, 538, 454, 435, 323, 295, 121, 112, 95, 85, 57.

B) *9α-chloro-11β,17α-dihydroxy-16α-methyl-21-thiol-1,4-pregnadiene-3,20-dione 17-(2'-furoate) 21-pivalate*

Prepare a suspension of 185 mg of the product of step A in 1.5 ml of glacial acetic acid. Place in a 2-dram vial, chill to 10° C and then add, under

stirring, 0.3 ml of a glacial acetic solution of hydrogenchloride (containing 10.9 mg of HCl/ml of acetic acid). Allow the contents of the stoppered vial to return to room temperature and then after 45 min pour into distilled water. Filter off the precipitate, wash with water, dilute sodiumcarbonate solution and then again with water. Dry overnight under air suction to give 185 mg of crude product.

Purify the crude product by preparative thin-layer chromatography on 1,000 μm silica gel plates using chloroform/ethylacetate (19:1) as eluant. Extract the desired band with ethylacetate, evaporate off the solvent and then extract the residue with a methylenechloride/diethylether/hexane mixture. Remove the solvent under air evaporation at room temperature and then dry under vacuum at 50° C to give 142 mg (yield 72% of the theory) of product. Recrystallize from a methylenechloride/hexane mixture at reflux temperature to give white needles. Dry under vacuum at 50° C to give 101 mg (yield 52% of the theory) of the title compound: λ max. 245 nm (methanol) (ε 26,330). The mass spectrum was consistent with the structure of the title compound (mol.wt. 603.155) as shown by peak matching; found: $m^+$556 (corresponding to loss of HCl: 36), $m^+$ 471 (corresponding to loss of $H_2C \cdot S \cdot CO \cdot t\text{-}Bu$:131).

*Example 17*

*9α-chloro-11β,17α,21-trihydroxy-16α-methyl-1,4-pregnadiene-3,20-dione 17-(2'-furoate) 21-methoxyacetate*

Prepare a solution of 480 mg of 17α,21-dihydroxy-16α-methyl-1,4,9(11)-pregnatriene-3,20-dione 17-(2'-furoate) 21-methoxyacetate in 9 ml of tetrahydrofuran. Cool to 0-2° C and under nitrogen add with stirring a solution of 0.17 ml of 70% perchloric acid in 0.42 ml of distilled water followed immediately thereafter by 127 mg of 1,3-dichloro-5,5-dimethylhydantoin. Stir for 5 min, then remove the ice-bath, discontinue the flow of nitrogen and allow the temperature to rise to room temperature. Allow to stand for 2 h, then pour the reaction mixture into an aqueous sodiumbisulfite solution (1.4 g of $NaHSO_3$ in 400 ml of distilled water). Add sodiumchloride to saturation. Filter off the solid product, wash with distilled water, partially air-dry and then complete drying at 60° C under vacuum.

Purify the crude product by preparative thin-layer chromatography on 1,000 μm silica gel plates using chloroform/ethylacetate (4:1) as eluant. Extract the desired band with ethylacetate, filter the extract, evaporate off the solvent and then dry the residue at 60° C under vacuum to give 240 mg of product.

Recrystallize from aqueous acetone at reflux temperature to give fine needles of the pure title product (yield 207 mg): λ max. 247 nm (ε 25,680); mass spectrum (no parent ion): 538, 473, 471, 443, 435, 426, 407, 389, 365, 363, 333, 331, 315, 313, 295, 277, 121, 95.

*Example 18*

*9α-chloro-11β,17α,21-trihydroxy-16α-methyl-1,4-pregnadiene-3,20-dione 17-(2'-furoate) 21-acetate*

Treat 615.6 mg of 17α,21-dihydroxy-16α-methyl-1,4,9(11)-pregnatriene-3,20-dione 17-(2'-furoate) 21-acetate in a similar manner to that described in Example 17 to give, after purification, 256 mg (yield 37.6% of the theory) of the pure title compound: λ max. 247 nm (ε 26,820); mass spectrum: 545,544, 473, 471, 333, 331, 315, 313, 295, 279, 277, 95, 43.

Alternatively, prepare the title compound using the procedure described in Example 16.

*Example 19*

*9α-fluoro-16α-methyl-11β,17α,21-triol-1,4-pregnadiene-3,20-dione 17-(2'-furoate) 21-acetate*

A) *9α-fluoro-16α-methyl-11β,17α,21-triol-1,4-pregnadiene-3,20-dione 11β-trifluoracetate 21-acetate*

Prepare a solution of 1.36 g of trifluoroacetic anhydride in 10 ml of pyridine. Add 5 ml of this reagent, chilled to 0-2° C, to a chilled solution of 434.5 mg of dexamethasoneacetate in pyridine. Stir for 15 min and then pour the resulting dard green solution into 200 ml of 3.6N sulfuric acid solution.

Filter off the green solid, wash with water, resuspend it in water, stir, filter again, wash and then dry under vacuum at room temperature. The crude product was purified by preparative thin-layer chromatography on 1,000 μm silica gel plates using chloroform/ethylacetate (9:1). The desired area was extracted with ethylacetate, the extract filtered and then evaporated at room temperature. The crude product was solidified with diethylether and hexane and then dried at 50° C under vacuum to give the title compound (198 mg; yield 37% of the theory).

B) *9α-fluoro-16α-methyl-11β,17α,21-tri-hydroxy-1,4-pregnadiene-3,20-dione 11β-trifluoroacetate 17-(2'-furoate) 21-acetate*

In a manner similar to that described in Example 2A treat, in methylenechloride, 150 mg of the product of step A with a reaction mixture of 2-furoylchloride and 4-dimethylaminopyridine, stirring the reaction mixture for 66 h. Dilute the reaction mixture with methylenechloride, wash with water, then 1N HCl followed by dilute sodiumcarbonate solution and then water adjusted to pH 5-6. Dry the methylenechloride solution over magnesiumsulfate, filter and then air-evaporate to give a residue of 162 mg. Purify by thin-layer chromatography on 1,000 μm silica gel plates using chloroform/ethylacetate as eluant as described in Example 2A. Dissolve the product on diethylether, filter and then evaporate. Dry at 50° C under vacuum to give 50 mg of product.

C) *9α-fluoro-16α-methyl-11β,17α,21-trihydroxy-1,4-pregnadiene-3,20-dione 17-(2'-furoate) 21-acetate*

Treat 21 mg of the product of step B with 72 mg of sodiumbenzoate in 2 ml of methanol for 3 h under stirring. Pour the reaction mixture into saturated aqueous sodiumchloride solution. Filter off the white precipitate, wash with water and then dry at room temperature.

Purify by treating with diethylether/hexane to give 25 mg of the title product which is identical to the product obtained in Example 3B(1) by reduction of a corresponding 11-ketone.

*Example 20*

*9α,21-dichloro-11β,17α-dihydroxy-16α-methyl-1,4-pregnadiene-3,20-dione 17-(2'-furoate)*

A) *21-chloro-9β,11β-epoxy-17α-hydroxy-16α-methyl-1,4-pregnadiene-3,20-dione*

Prepare a solution of 5.0 g of 9β,11β-epoxy-17α,21-dihydroxy-16α-methyl-1,4-pregnadiene-3,20-dione in 20 ml of dry pyridine. Cool on an ice-bath; to the stirred solution under nitrogen, add dropwise 1.1 ml of mesylchloride. Remove the ice-bath and continue stirring at room temperature for 30 min. Add 2.0 g of lithiumchloride and continue stirring for a further 150 min. Add to a mixture of 150 ml of ethylacetate and 100 ml of distilled water in a separating tunnel. Wash the organic phase with diluted 3% aqueous hydrochloric acid, then saturated aqueous sodiumchloride solution and finally saturated aqueous sodiumbicarbonate solution. Dry the organic phase over magnesiumsulfate, filter and remove the solvent. Recrystallize from methylenechloride/diethylether to give from the combined crops 4.62 g of the title compound.

B) *21-chloro-9β,11β-epoxy-17α-hydroxy-16α-methyl-1,4-pregnadiene-3,20-dione 17-(2'-furoate)*

Prepare under argon a solution of 8 g of 4-dimethylaminopyridine in 250 ml of dry methylenechloride. Cool on an ice-bath and add to the stirred solution 6.0 ml of 2-furoylchloride. Remove from the ice-bath, allow the temperature to rise to room temperature and then add 11.5 g of the product of step A. After 24 h, add under rapid stirring 500 ml of ethylacetate saturated with water. Filter off the precipitate and then evaporate off the solvent from the filtrate to give the crude title product which was used without further purification in step C.

C) *9α,21-dichloro-11β,17α-dihydroxy-16α-methyl-1,4-pregnadiene-3,20-dione 17-(2'-furoate)*

To the product of step B add 50 ml of glacial acetic acid. To the stirred solution under argon then add a solution of 3.5 g of anhydrous hydrogenchloride in 125 ml of glacial acetic acid. Stir for 15 min and then quench with 500 ml of distilled water. Filter off the solids, recrystallize from methanol/water, dry for 24 h under vacuum to give 12.6 g of the title compound (yield 83% of the theory) essentially identical with the product obtained in Example 14.

Using the procedures described in the foregoing examples, the following compounds of the general Formula I may be prepared:

(*a*) 9α,11β-dichloro-16α-methyl-1,4-pregnadiene-17α,21-diol-3,20-dione 17-(2'-furoate) 21-butyrate: λ max. 245 nm (ε 23,600) 245-258 nm (broad); mass spectrum (no parent ion): 491, 489, 373, 371, 351, 349, 331, 279, 95, 91, 43;

(*b*) 9α-fluoro-16α-methyl-1,4-pregnadiene-11β,17α,21-triol-3,20-dione 17-(2'-furoate) 21-butyrate: λ max. 247 nm (ε 26,390); mass spectrum (no parent ion): 536, 456, 455, 315, 295, 95, 71;

(*c*) 9α,11β-dichloro-16α-methyl-1,4-pregnadiene-17α,21-diol-3,20-dione 17-(2'-furoate): λ max. 243 nm (ε 20,910) (inflexions at 247, 251 and 256 nm); mass spectrum (no parent ion): 491, 489, 351, 349, 315, 279, 95;

(*d*) 9α,11β-dichloro-16α-methyl-1,4-pregnadiene-17α,21-diol-3,20-dione 17-(3'-furoate): λ max. 236 nm (ε 17,400) (inflexions at 255, 262 and 268 nm); mass spectrum (no parent ion): 491, 489, 410, 408, 389, 379, 377, 351, 349, 338, 306, 279, 95;

(*e*) 9α,11β,21-trichloro-16β-methyl-1,4-pregnadiene-17α-ol-3,20-dione 17-(2'-furoate): λ max. 245 nm (ε 23,420), 254 nm (ε 22,950); mass spectrum (no parent ion): 491, 489, 351, 349, 315, 313, 279, 277, 95;

(*f*) 9α,11β-dichloro-21-fluoro-16α-methyl-1,4-pregnadiene-17α-ol-3,20-dione 17-(2'-furoate): λ max. 245 nm (ε 22,760); mass spectrum (no parent ion): 488, 486, 451, 390, 376, 374, 351, 349, 340, 325, 315, 313, 279, 277, 95;

(*g*) 9α-fluoro-16-methylene-1,4-pregnadiene-11β,17α,21-triol-3,20-dione 17-(2'-furoate) 21-acetate: λ max. 245 nm (ε 25,270); mass spectrum: 526, 506, 415, 313, 112, 95;

(*h*) 9α,21-dichloro-16α-methyl-1,4-pregnadiene-11β,17α-diol-3,20-dione 17-(2'-thenoate): λ max. 243 nm (ε 23,500) (inflexions at 262 and 272); mass spectrum (no parent ion): 502, 500, 451, 423, 295, 111, 91;

(*i*) 9α-chloro-16-methylene-1,4-pregnadiene-11β,17α,21-triol-3,20-dione 17-(2'-furoate) 21-acetate: λ max. 246 nm (ε 24,370); mass spectrum: 542, 506, 469, 431, 295, 293, 111, 95; and

(*j*) 9α-fluoro-21-chloro-16α-methyl-1,4-pregnadiene-17α-ol-3,11,20-trione 17-(3'-furoate): λ max. 237 nm (ε 18,570); mass spectrum: 504, 502, 453, 425, 405, 281, 96, 95.

As previously mentioned, the compounds of the general Formula I exhibit useful corticosteroid activity, in particular useful anti-inflammatory activity. Representative 3,20-dioxo-1,4-pregnadiene-17α-ol 17-aromatic heterocyclic carboxylates of the general Formula I have been found to exhibit unexpectedly enhanced anti-inflammatory activity compared with known high-potency reference steroidal esters. Thus, when tested in mice by a modification of the well-known 5-d croton oil ear edema tests (B. N. Lutsky, *et al.*, "Arzneim.-Forsch.", 29, 992, 1979), 9α,11β,21-trichloro-16α-methyl-1,4-pregnadiene-17α-ol-3,20-dione 17-(2'-furoate) exhibited topical potency about eight times that of the well-known topical anti-inflammatory agent betamethasone 17-valerate.

In another of its aspects the invention provides pharmaceutical compositions suitable especially for the treatment of inflammatory conditions in animals and humans and comprising a compound of the general Formula I together with a suitable pharmaceutical carrier. In general, the compounds of the Formula I may be formulated in a manner similar to that for the corresponding known 17-alkanoates.

For topical or local administration the compounds of the Formula I may be in the form of creams, lotions, aerosols, ointments or powders in the treatment of all corticosteroid responsive dermatoses such as contact and allergic dermatitis, eczemas and psoriasis or may be in the form of ophthalmic suspensions or nasal sprays.

Ointments and creams may be formulated in conventional manner with an aqueous or oily base with the addition of suitable thickening and/or gelling agents.

Lotions, similarly, may be formulated with an aqueous or oily base and may include in conventional manner stabilizing agents, emulsifying agents, dispersing agents, suspending agents, thickening agents, coloring agents, perfumes and the like.

Powders may be formulated using any suitable powder base, e.g. talc, lactose, starch, etc. Drops may be formulated with an aqueous base or non-aqueous base also comprising one or more dispersing agents, suspending agents or solubilizing agents.

The pharmaceutical compositions may include one or more preservatives or bacteriostatic agents.

The compositions may also contain other active ingredients such as antimicrobial agents, particularly antibiotics.

The proportion of active steroid in the compositions according to the invention depends on the precise type of formulation to be prepared but will generally be within the range of from 0.0001 to 5% by weight. Generally, however, for most types of preparations advantageously the proportion used will be within the range of from 0.001 to 0.5% and preferably 0.01 to 0.25%.

The daily dosage of the indicated pharmaceutical composition will, as for compositions containing the corresponding 17-alkanoates, be determined by the attending physician and will depend upon such factors as the nature and severity of the inflammation being treated, the age and size of the patient and the specific potency of the Formula I compound being administered.

The following examples illustrate topical formulations in accordance with the invention. In each, a preferred active ingredient is:

9α,11β,21-trichloro-16α-methyl-1,4-pregnadiene-17α-ol-3,20-dione 17-(2'-furoate); however, this compound may be replaced by equivalent quantities of other compounds of the Formula I, such as:

9α,11β-dichloro-16α-methyl-1,4-pregnadiene-17α,21-diol-3,20-dione 17-(2'-furoate) 21-acetate,

9α-fluoro-16α-methyl-1,4-pregnadiene-11β,17α,21-triol-3,20-dione 17-(2'-furoate) 21-acetate,

9α-fluoro-21-chloro-16α-methyl-1,4-pregnadiene-11β,17α-diol-3,20-dione 17-(2'-furoate), and

9α-21-dichloro-16α-methyl-1,4-pregnadiene-11β,17α-diol-3,20-dione 17-(2'-furoate).

*Formulation examples*

1. *Glycol Ointment*

| | *mg/g* |
|---|---|
| Active ingredient | 0.1-5.0 |
| Hexylene glycol | 100.0 |
| Propylene glycol monostearate | 20.0 |
| White wax | 60.0 |
| White petrolatum to make | 1.00 g |

Melt and heat together to 60-65° C the propylene glycol monostearate, white wax and white petrolatum. Heat the hexylene glycol to 40° C and dissolve the active ingredient in it. Add, with agitation, the solution of the hexylene glycol to the above oily phase (cooled to 55° C). Cool, with agitation, until the temperature reaches 30° C.

2. *Lotion*

| | *mg/g* |
|---|---|
| Active ingredient | 0.1-5.0 |
| Ethylalcohol | 400.0 |
| Polyethylene glycol 400 | 300.0 |
| Hydroxypropyl cellulose | 5.0 |
| Propylene glycol to make | 1.0 g |

Dissolve the active ingredient in the mixture of the ethylalcohol, polyethylene glycol and propylene glycol. Slowly add the hydroxypropyl cellulose and continue to agitate until the hydroxypropyl cellulose is completely dispersed and wetted and a clear lotion is produced.

3. *Cream*

| | *mg/g* |
|---|---|
| Active ingredient | 0.1-5.0 |
| Isopropyl palmitate | 100.0 |
| Glyceryl stearate | 80.0 |
| Promulgen-Type D (Robinson, Wagner Co.) | 50.0 |
| White wax | 50.0 |
| Propylene glycol | 100.0 |
| Purified water to make | 1.00 g |

Melt together and heat to 75° C the white wax, glyceryl stearate, Promulgen-Type D and a portion of the isopropyl palmitate and maintain the temperature. Disperse the active ingredient in the

remaining portion of the isopropyl palmitate and mill the dispersion. While agitating add the dispersion to the above oily phase. Heat together the water and the propylene glycol to 75° C. Add the solution to the above oily phase with agitation. Cool, with agitation, until the temperature reaches 30° C.

4. *Topical Aerosol*

| | *mg/can* |
|---|---|
| Active ingredient | 6.4 |
| Mineral oil | 1,250.0 |
| Neobee M-5 (Caprylic/Capric Glyceride) (PVO International, Inc.) | 3,743.6 |
| Dichlorodifluoromethane | 17,200.0 |
| Trichloromonofluoromethane | 68,888.0 |
| | 91,000.0 |

Dissolve the active ingredient in Neobee M-5 and add mineral oil. Place this concentrate into an aerosol and crimp a valve on the can. Inject the dichlorodifluoromethane and trichloromonofluoromethane mixture into the container through the valve.

The compounds of the general Formula I are relatively non-toxic, at least not significantly more toxic than comparable 17 non-heterocyclic carboxylate derivatives corresponding to Formula I such as betamethasone 17-valerate, with the ratio of potency to toxicity being generally better than betamethasone 17-valerate.

**Claims** for the Contracting States BE, CH, DE, FR, GB, IT, LI, NL, SE

1. 3,20-dioxo-1,4-pregnadiene-17α-ol 17-atomatic heterocyclic carboxylates of the general Formula I:

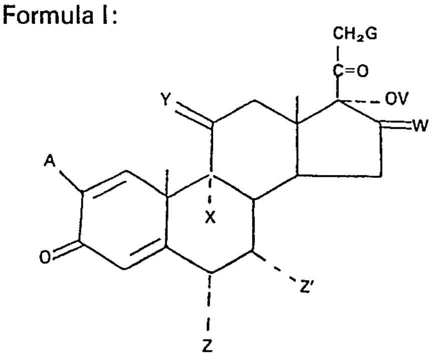

(I)

wherein

A is hydrogen or, provided Y is (H,β-OH), A may also be chloro, fluoro or methyl;

X is hydrogen or a halogen atom having an atomic weight less than 100;

Y is oxygen, (H,β-OH) or, provided that X is hydrogen,

Y may also be (H,H) or, provided X is chloro or bromo, Y may also be (H,β-halogen), the β-halogen having an atomic weight of less than 100 and being at least as electronegative as X;

Z is hydrogen, methyl, chloro or fluoro;

Z' is hydrogen or, provided that Z is hydrogen, may also be halogen having an atomic weight of less than 100;

V is an acyl radical of a thiophenecarboxylic acid, pyrrolecarboxylic acid or furancarboxylic acid or the methyl- or halogen-substituted derivatives thereof;

W is (H,H), (H, $C_{1-6}$ alkyl) or (H, $\alpha OV_1$) where $V_1$ is hydrogen or an acyl radical of retinoic acid or a carboxylic acid having up to 12 carbon atoms; or W is =CHT where T is hydrogen, $C_{1-6}$ alkyl, fluoro or chloro, and

G is hydrogen, a halogen atom having an atomic weight less than 100 or $-QV_2$ where Q is an oxygen or sulfur atom and $V_2$ is as defined for V or $V_1$ or is an acyl radical of phosphoric acid which may be in the form of a mono- or dialkali metal or an alkaline earth metal salt thereof;

and the 6-dehydro and 1,2-dihydro derivatives of the foregoing.

2. A compound as claimed in claim 1, characterized by being a 3,20-dioxo-1,4-pregnadiene.

3. A compound as claimed in claim 1 or 2, characterized in that V is furancarbonyl or thiophenecarbonyl.

4. A compound as claimed in any one of claims 1 to 3, characterized by being of the general Formula II:

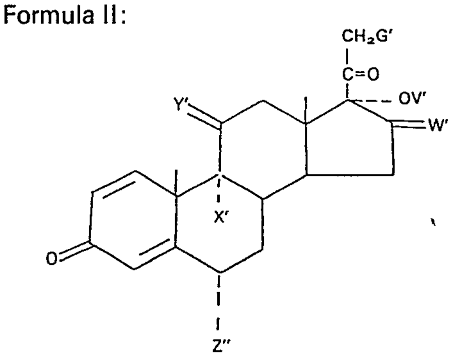

(II)

where

X' is fluoro or chloro;

Y' is (H,β-OH) or, provided that X' is chloro, Y' may also be (H,β-halogen) the β-halogen having an atomic weight of less than 100 and being at least as electronegative as X';

Z'' is hydrogen or fluoro;

W' is (H,H) or (H,$CH_3$);

V' is furancarbonyl or thiophenecarbonyl, and

G' is chloro or fluoro or $-QV'_2$, where Q is an oxygen or sulfur atom (preferably oxygen) and $V'_2$ is hydrogen or an acyl radical of retinoic acid, of a carboxylic acid having up to 12 (preferably up to 8) carbon atoms or of phosphoric acid which may be in the form of a mono- or dialkali metal or alkaline earth metal salt.

5. A compound as claimed in claim 4, characterized in that G' is chloro, $C_{1-6}$ alkanoyloxy or $C_{1-6}$ alkoxy $C_{1-6}$ alkanoyloxy (preferably acetoxy or methoxyacetoxy), X' is chloro and Y' is (H,β-Cl) or X' is chloro or fluoro and Y' is (H,β-OH) and W' is (H,H) or (H, $CH_3$), preferably (H, α-$CH_3$).

6. A compound as claimed in claim 1, said compound being selected from:

9α, 11β, 21-trichloro-16α-methyl-1,4-pregnadiene-17α-ol-3,20-dione 17-(2'-furoate);

6α-fluoro-9α, 11β, 21-trichloro-16α-methyl-1,4-pregnadiene-17α-ol-3,20-dione 17-(2'-furoate);

9α-fluoro-16α-methyl-1,4-pregnadiene-11β, 17α, 21-triol-3,20-dione 17-(2'-furoate) 21-acetate;

9α-fluoro-16α-methyl-1,4-pregnadiene-11β, 17α,21-triol-3,20-dione 17-(2'-furoate) 21-methoxyacetate;

9α-fluoro-16α-methyl-1,4-pregnadiene-11β, 17α,21-triol-3,20-dione 17-(3'-furoate) 21-acetate;

9α-fluoro-21-chloro-16α-methyl-1,4-pregnadiene-11β,17α-diol-3,20-dione 17-(2'-furoate);

9α,21-dichloro-16α-methyl-1,4-pregnadiene-11β,17α-diol-3,20-dione 17-(2'-furoate);

9α-chloro-16α-methyl-1,4-pregnadiene-11β,17α,21-triol-3,20-dione 17-(2'-furoate) 21-acetate;

9α-chloro-16α-methyl-1,4-pregnadiene-11β, 17α,21-triol-3,20-dione 17-(2'-furoate) 21-methoxyacetate;

9α-fluoro-21-fluoro-16α-methyl-1,4-pregnadiene-11β,17α-diol-3,20-dione 17-(2'-furoate), and

9α-chloro-16α-methyl-1,4-pregnadiene-11β, 17α-diol-21-thiol-3,20-dione 17-(2'-furoate) 21-pivalate.

7. A compound as claimed in claim 1, said compound being selected from:

9α, 11β, 21-trichloro-16α-methyl-1,4-pregnadiene-17α-ol-3,20-dione 17-(2'-thenoate);

9α-fluoro-16α-methyl-1,4-pregnadiene-11β, 17α, 21-triol-3,20-dione 17-(2'-thenoate) 21-acetate;

9α-fluoro-21-chloro-16α-methyl-1,4-pregnadiene-11β,17α-diol-3,20-dione 17-(2'-furoate);

9α,21-dichloro-16α-methyl-1,4-pregnadiene-11β,17α-diol-3,20-dione 17-(2'-theonate).

8. A process for the preparation of a 17α-aromatic heterocyclic carboxylate claimed in claim 1, said process being characterized in that an appropriate 3,20-dioxo-1,4-pregnadiene starting material, or a 6-dehydro or 1,2-dihydro derivative thereof, is subjected to:

A: introduction of the desired ester group at the 16α,17α and/or 21 positions, or

B: hydrolysis of an ester group present at one or more of positions 11β,16α and 21 or of a 16α,17α- or 17α,21-orthoester group, or

C: halogenation at one or more of positions 9α,11β and 21, or

D: reduction of an 11-oxo group to an 11β-hydroxy group.

9. Process as claimed in claim 8 process A, characterized by esterifying a 3,20-dioxo-1,4-pregnadiene-17α,21-diol 21-acylate otherwise corresponding to that of Formula I wherein A and Z' are both hydrogen, X is chloro and Y is (H,β-Cl), or X is fluoro and Y is (H,β-OH), Z is hydrogen or fluoro, W is ($H,CH_3$) preferably ($H,\alpha-CH_3$) and G is an acyloxy radical as defined in the definition of $-QV_2$, with a reactive derivative of furan- or thiophenecarboxylic acid.

10. A process as claimed in claim 8 process B, characterized by hydrolyzing at position 21 a 17α-furoate 21-acylate or 17α-thenoate 21-acylate or a 17α,21-orthoester, that is a 17α,21-alkylorthofuroate or -theonate, otherwise corresponding to that of Formula I wherein A and Z' are both hydrogen, X and Y are as defined for Formula I, Z is hydrogen or fluoro, and W is (H, $CH_3$), preferably ($H,\alpha-CH_3$) and where desired re-esterifying at C-21 a so-obtained 17α,21-diol 17α-furoate or -thenoate to give another required 17α,21-diester of the general Formula I.

11. A process as claimed in claim 8 process C, characterized by chlorinating at the C-21 position a 3,20-dioxo-1,4-pregnadiene 21-reactive acylate, such as 21-sulfonate, otherwise corresponding to that of the general Formula I wherein A and Z' are both hydrogen, X is fluoro, Y is (H,β-OH), Z is hydrogen or fluoro, W is ($H,CH_3$) preferably ($H,\alpha-CH_3$) and V is an acyl radical of furan- or thiophenecarboxylic acid, with a suitable source of chloride ion.

12. A process as claimed in claim 8 process C, characterized by addition of 2 chlorine atoms, or a 9α-chloro and 11β-hydroxy group, to the 9(11)-double bond of a 3,20-dioxo-1,4, 9(11)-pregnatriene, otherwise corresponding to that of Formula I wherein A and Z' are both hydrogen, Z is hydrogen or fluoro, W is ($H,CH_3$), preferably ($H,\alpha-CH_3$), G is chloro, fluoro or an acyloxy radical as defined in the definition of $-QV_2$ and V is an acyl radical of furan- or thiophenecarboxylic acid to give the required 9α,11β-dichloro- and 9α-chloro-11β-hydroxy compounds of the general Formula I, and where desired converting a 21-acylate function into the corresponding 21-chloro function.

13. A process as claimed in claim 8 process C, characterized by ring opening of the 9β,11β-epoxide ring, with concomitant addition of HF or HCl, of a 9β,11β-oxido-3,20-dione-1,4-pregnadiene otherwise corresponding to that of the Formula I wherein A and Z' are both hydrogen, Z is hydrogen or fluoro, W is ($H,CH_3$), preferably ($H,\alpha-CH_3$), G is chloro, fluoro or an acyloxy radical as defined in the definition of $-QV_2$ and V is an acyl radical of furan- or thiophenecarboxylic acid to give the required 9α-chloro-11β-hydroxy- or 9α-fluoro-11β-hydroxy compound of the general Formula I.

14. A process as claimed in claim 8 process D, characterized by reducing at the C-11 position a 3,11,20-trioxo-1,4-pregnadiene corresponding to that of the general Formula I wherein A and Z' are both hydrogen, X is hydrogen or fluoro, Z is hydrogen or fluoro, W is ($H,CH_3$), preferably ($H,\alpha-CH_3$), V is an acyl radical of furan- or thiophenecarboxylic acid and G is an acyloxy radical as defined in the definition of $-QV_2$.

15. A compound as claimed in any one of claims 1 to 7 for use as a pharmaceutical, especially for use as an anti-inflammatory.

16. A pharmaceutical composition, especially for use as an anti-inflammatory, comprising as active ingredient a compound as claimed in any

one of claims 1 to 7 together with a suitable pharmaceutical carrier.

**Claims** of the Contracting State: AT

1. A process for the preparation of 3,20-dioxo-1,4-pregnadiene-17α-ol 17-aromatic heterocyclic carboxylates of the general Formula (I):

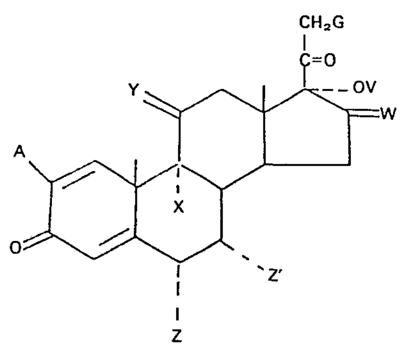

(I)

wherein

A is hydrogen or, provided Y is (H,βOH), A may also be chloro, fluoro or methyl;

X is hydrogen or a halogen atom having an atomic weight less than 100;

Y is oxygen, (H,β-OH) or, provided that X is hydrogen,

Y may also be (H,H) or, provided X is chloro or bromo, Y may also be (H,β-halogen), the β-halogen having an atomic weight of less than 100 and being at least as electronegative as X;

Z is hydrogen, methyl, chloro or fluoro;

Z' is hydrogen or, provided that Z is hydrogen, may also be halogen having an atomic weight of less than 100;

V is an acyl radical of a thiophenecarboxylic acid, pyrrolecarboxylic acid or furancarboxylic acid or the methyl- or halogen-substituted derivatives thereof;

W is (H,H), (H, $C_{1-6}$ alkyl) or (H, $\alpha OV_1$) where $V_1$ is hydrogen or an acyl radical of retinoic acid or a carboxylic acid having up to 12 carbon atoms, or W is =CHT where T is hydrogen, $C_{1-6}$ alkyl, fluoro or chloro, and

G is hydrogen, a halogen atom having an atomic weight less than 100 or $-QV_2$ where Q is an oxygen or sulfur atom and $V_2$ is as defined for V or $V_1$ or is an acyl radical of phosphoric acid which may be in the form of a mono- or dialkali metal or an alkaline earth metal salt thereof;

and the 6-dehydro and 1,2-dihydro derivatives of the foregoing, characterized by comprising one or more of the following processes A to D:

A: introduction of a desired ester group at one or more of positions 16α,17α and/or 21 of a starting material otherwise corresponding to that of the general Formula (I);

B: hydrolysis of an ester group present at one or more of positions 11β, 16α and 21 in a starting material otherwise corresponding to that of the general Formula (I);

C: halogenation at one or more of positions 9α, 11β and 21 of a 9(11)-ene, a 9β,11β-epoxide or a 21-reactive acylate starting material to give a corresponding 9α,11β-dihalo, 9α-halo-11β-hydroxy or 21-halo compound of the general Formula (I), and

D: reduction at the 11-keto group of an 11-one starting material to give a corresponding 11β-hydroxy compound of the general Formula (I).

2. A process as claimed in claim 1 process A, characterized by esterifying a 3,20-dioxo-1,4-pregnadiene 17α,21-diol 21-acylate otherwise corresponding to that of Formula (I) wherein A and Z' are both hydrogen, X is chloro and Y is (H,β-Cl), or X is fluoro and Y is (H,β-OH), Z is hydrogen or fluoro, W is (H,$CH_3$), preferably (H,α-$CH_3$), and G is an acyloxy radical as defined in the definition of $-QV_2$, with a reactive derivative of furan- or thiophenecarboxylic acid.

3. A process as claimed in claim 1 process B, characterized by hydrolyzing at position 21 a 17α-furoate-21-acylate or 17α-thenoate-21-acylate or a 17α,21-orthoester, that is a 17α,21-alkylorthofuroate or -thenoate, otherwise corresponding to that of Formula (I) wherein A and Z' are both hydrogen, X and Y are as defined for Formula (I), Z is hydrogen or fluoro, and W is (H,$CH_3$) preferably (H,α-$CH_3$), and where desired re-esterifying at C-21 a so-obtained 17α,21-diol 17α-furoate or -thenoate to give another required 17α,21-diester of the general Formula (I).

4. A process as claimed in claim 1 process C, characterized by chlorinating at the C-21 position a 3,20-dioxo-1,4-pregnadiene 21-reactive acylate, such as 21-sulfonate, otherwise corresponding to that of the general Formula (I) wherein A and Z' are both hydrogen, X is fluoro, Y is (H,β-OH), Z is hydrogen or fluoro, W is (H,$CH_3$), preferably (H,α-$CH_3$), and V is an acyl radical of furan- or thiophenecarboxylic acid, with a suitable source of chloride ion.

5. A process as claimed in claim 1 process C, characterized by addition of two chlorine atoms, or a 9α-chloro and 11β-hydroxy group, to the 9(11)-double bond of a 3,20-dioxo-1,4,9(11)-pregnatriene otherwise corresponding to that of Formula (I) wherein A and Z' are both hydrogen, Z is hydrogen or fluoro, W is (H,$CH_3$) preferably (H,α-$CH_3$), G is chloro, fluoro or an acyloxy radical as defined in the definition of $-QV_2$, and V is an acyl radical of furan- or thiophenecarboxylic acid to give the required 9α,11β-dichloro- and 9α-chloro-11β-hydroxy compounds of the general Formula (I), and where desired converting a 21-acylate function into the corresponding 21-chloro function.

6. A process as claimed in claim 1 process C, characterized by ring opening of the 9β,11β-epoxide ring, with concomitant addition of HF or HCl of a 9β,11β-oxido-3,20-dione-1,4-pregnadiene otherwise corresponding to that of the Formula (I) wherein A and Z' are both hydrogen, Z is hydrogen or fluoro, W is (H,$CH_3$), preferably (H,α-$CH_3$), G is chloro, fluoro or an acyloxy radical as defined in the definition of $-QV_2$ and V is an acyl radical of furan- or thiophenecarboxylic acid to give the required 9α-chloro-11β-hydroxy

or 9α-fluoro-11β-hydroxy compound of the general Formula (I).

7. A process as claimed in claim 1 process D, characterized by reducing at the C-11 position a 3,11,20-trioxo-1,4-pregnadiene corresponding to that of the general Formula (I) wherein A and Z' are both hydrogen, X is hydrogen or fluoro, Z is hydrogen or fluoro, W is $(H,CH_3)$, preferably $(H,\alpha\text{-}CH_3)$, V is an acyl radical or furan- or thiophenecarboxylic acid and G is an acyloxy radical as defined in the definition of $-QV_2$.

8. A process as claimed in claim 1 process B, characterized by hydrolyzing at position 11 an 11β-protected-hydroxy-17α-furoate (or 17α-thenoate) 21-acylate otherwise corresponding to that of Formula (I) wherein A and Z' are both hydrogen, X is halogen preferably fluoro, Z is hydrogen or fluoro, W is $(H,CH_3)$ preferably $(H,\alpha\text{-}CH_3)$, and the 21-acylate is the group $-QV_2$ where Q and $V_2$ are as defined for Formula (I) to give the corresponding 11β-free-hydroxy-17α,21-diester of the general Formula (I).

9. A process for the preparation of a pharmaceutical composition which comprises admixing a compound of the general Formula (I) set forth in claim 1 with a suitable pharmaceutically acceptable carrier.

10. A process as claimed in claim 9, wherein the compound of the general Formula (I) set forth in claim 1 has been prepared by a process as claimed in any one of claims 1 to 8.

**Patentansprüche** für die Vertragsstaaten BE, CH, DE, FR, GB, IT, LI, NL, SE

1. 3,20-Dioxo-1,4-pregnadien-17α-ol-17-aromatisch-heterocyclische Carboxylate der allgemeinen Formel (I):

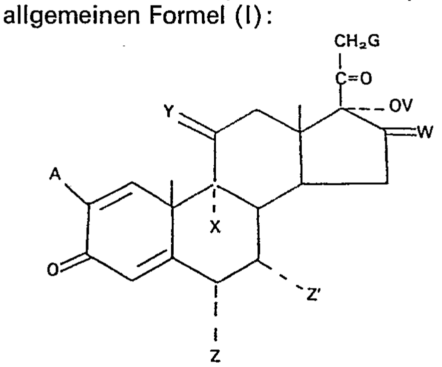

(I)

in der

A Wasserstoff ist oder, vorausgesetzt, dass Y (H,β-OH) ist, auch Chloro, Fluoro oder Methyl sein kann;

X Wasserstoff oder ein Halogenatom mit einem Atomgewicht von weniger als 100 ist;

Y Sauerstoff oder (H,β-OH) ist oder, vorausgesetzt, dass X Wasserstoff ist, auch (H,H) sein kann oder, vorausgesetzt, dass X Chloro oder Bromo ist, auch (H, β-Halogen) sein kann, wobei das β-Halogen ein Atomgewicht von weniger als 100 besitzt und wenigstens so elektronegativ wie X ist;

Z Wasserstoff, Methyl, Chloro oder Fluoro ist;

Z' Wasserstoff ist oder, vorausgesetzt, dass Z Wasserstoff ist, auch Halogen mit einem Atomgewicht von weniger als 100 sein kann;

V ein Acylrest einer Thiophencarbonsäure, Pyrrolcarbonsäure oder Furancarbonsäure oder eines davon abgeleiteten methyl- oder halogensubstituierten Derivats ist;

W (H,H), $(H,C_{1\text{-}6}\text{-Alkyl})$ oder $(H,\alpha OV_1)$ ist, worin $V_1$ Wasserstoff oder ein Acylrest der Retinasäure (Vitamin-A-Säure) oder einer Carbonsäure mit bis zu 12 Kohlenstoffatomen ist, oder W = CHT ist, worin T Wasserstoff, $C_{1\text{-}6}$-Alkyl, Fluoro oder Chloro ist, und

G Wasserstoff, ein Halogenatom mit einem Atomgewicht von weniger als 100 oder $-QV_2$ ist, worin Q ein Sauerstoff- oder Schwefelatom ist, und $V_2$ die für V oder $V_1$ angegebene Bedeutung hat oder ein Acylrest der Phosphorsäure ist, der in Form eines Mono- oder Dialkalimetallsalzes oder eines Erdalkalimetallsalzes derselben vorliegen kann,

sowie die 6-Dehydro- und 1,2-Dihydroderivate der vorgenannten Verbindungen.

2. Verbindung nach Anspruch 1, dadurch gekennzeichnet, dass sie 3,20-Dioxo-1,4-pregnadien ist.

3. Verbindung nach einem der beiden Ansprüche 1 oder 2, dadurch gekennzeichnet, dass V Furancarbonyl oder Thiophencarbonyl ist.

4. Verbindung nach irgendeinem der Ansprüche 1 bis 3, dadurch gekennzeichnet, dass sie eine solche der allgemeinen Formel (II) ist:

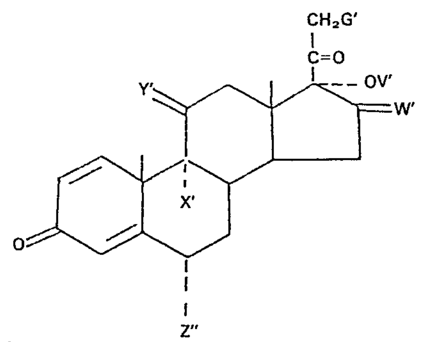

(II)

in der

X' Fluoro oder Chloro ist;

Y' (H,β-OH) ist oder, vorausgesetzt, dass X' Chloro ist, auch (H,β-Halogen) sein kann, wobei das β-Halogen ein Atomgewicht von weniger als 100 besitzt und wenigstens so elektronegativ wie X' ist;

Z'' Wasserstoff oder Fluoro ist;

W' (H,H) oder $(H,CH_3)$ ist;

V' Furancarbonyl oder Thiophencarbonyl ist, und

G' Chloro oder Fluoro oder $-QV'_2$ ist, worin Q ein Sauerstoff- oder Schwefelatom (vorzugsweise Sauerstoff) ist, und $V'_2$ Wasserstoff oder ein Acylrest der Retinasäure (Vitamin-A-Säure), einer Carbonsäure mit bis zu 12 (vorzugsweise bis zu 8) Kohlenstoffatomen oder der Phosphorsäure ist, der in Form eines Mono- oder Dialkalimetallsalzes oder eines Erdalkalimetallsalzes derselben vorliegen kann.

5. Verbindung nach Anspruch 4, dadurch gekennzeichnet, dass

G' Chloro, $C_{1-6}$-Alkanoyloxy oder $C_{1-6}$-Alkoxy-$C_{1-16}$-alkanoyloxy (vorzugsweise Acetoxy oder Methoxyacetoxy) ist;

X' Chloro ist und Y' (H,β-Cl) ist oder

X' Chloro oder Fluoro ist und Y' (H,β-OH) ist, und

W' (H,H) oder ($H,CH_3$), vorzugsweise (H,α-$CH_3$), ist.

6. Verbindung nach Anspruch 1, dadurch gekennzeichnet, dass sie ausgewählt ist aus:

9α,11β,21-Trichloro-16α-methyl-1,4-pregnadien-17α-ol-3,20-dion-17-(2'-furoat);

6α-Fluoro-9α,11β,21-trichloro-16α-methyl-1,4-pregnadien-17α-ol-3,20-dion-17-(2'-furoat);

9α-Fluoro-16α-methyl-1,4-pregnadien-11β, 17α,21-triol-3,20-dion-17-(2'-furoat)-21-acetat;

9α-Fluoro-16α-methyl-1,4-pregnadien-11β, 17α,21-triol-3,20-dion-17-(2'-furoat)-21-methoxyacetat;

9α-Fluoro-16α-methyl-1,4-pregnadien-11β, 17α,21-triol-3,20-dion-17-(3'-furoat)-21-acetat;

9α-Fluoro-21-chloro-16α-methyl-1,4-pregnadien-11β,17α-diol-3,20-dion-17-(2'-furoat);

9α,21-Dichloro-16α-methyl-1,4-pregnadien-11β,17α-diol-3,20-dion-17-(2'-furoat);

9α-Chloro-16α-methyl-1,4-pregnadien-11β, 17α,21-triol-3,20-dion-17-(2'-furoat)-21-acetat;

9α-Chloro-16α-methyl-1,4-pregnadien-11β, 17α,21-triol-3,20-dion-17-(2'-furoat)-21-methoxyacetat;

9α-Fluoro-21-fluoro-16α-methyl-1,4-pregnadien-11β,17α-diol-3,20-dion-17-(2'-furoat), und

9α-Chloro-16α-methyl-1,4-pregnadien-11β, 17α-diol-21-thiol-3,20-dion-17-(2'-furoat)-21-pivalat.

7. Verbindung nach Anspruch 1, dadurch gekennzeichnet, dass sie ausgewählt ist aus:

9α,11β,21-Trichloro-16α-methyl-1,4-pregnadien-17α-ol-3,20-dion-17-(2'-thenoat);

9α-Fluoro-16-methyl-1,4-pregnadien-11β, 17α,21-triol-3,20-dion-17-(2'-thenoat)-21-acetat;

9α-Fluoro-21-chloro-16α-methyl-1,4-pregnadien-11β,17α-diol-3,20-dion-17-(2'-thenoat), und

9α,21-Dichloro-16α-methyl-1,4-pregnadien-11β,17α-diol-3,20-dion-17-(2'-thenoat).

8. Verfahren zur Herstellung eines 17α-aromatisch-heterocyclischen Carboxylats nach Anspruch 1, dadurch gekennzeichnet, dass ein geeigneter 3,20-Dioxo-1,4-pregnadienausgangsstoff oder ein 6-Dehydro- oder 1,2-Dihydroderivat desselben unterworfen wird:

A: der Einführung der gewünschten Estergruppe in der 16α-, 17α- und/oder 21-Stellung; oder

B: der Hydrolyse einer in einer oder mehrerer der 11β-, 16α- und 21-Stellungen vorhandenen Estergruppe oder einer 16α,17α- oder 17α,21-Orthoestergruppe; oder

C: der Halogenierung in einer oder mehrerer der Stellungen 9α, 11β und 21; oder

D: der Reduktion einer 11-Oxogruppe zu einer 11β-Hydroxygruppe.

9. Verfahren nach Anspruch 8, Verfahren A, dadurch gekennzeichnet, dass ein 3,20-Dioxo-1,4-pregnadien-17α,21-diol-21-acylat, das ansonsten einem solchen der Formel (I) entspricht, in der A und Z' beide Wasserstoff sind, X Chloro ist und Y (H,β-Cl) ist, oder X Fluoro ist und Y (H,β-OH) ist, Z Wasserstoff oder Fluoro ist, W ($H,CH_3$), vorzugsweise (H,α-$CH_3$), ist und G ein Acyloxyrest ist, wie er in der Definition für $-QV_2$ festgelegt ist, mit einem reaktionsfähigen Derivat der Furan- oder Thiophencarbonsäure verestert wird.

10. Verfahren nach Anspruch 8, Verfahren B, dadurch gekennzeichnet, dass ein 17α-Furoat-21-acylat oder 17α-Thenoat-21-acylat oder ein 17α,21-Orthoester, das heisst, ein 17α,21-Alkylorthofuroat oder -thenoat, das ansonsten einem solchen der Formel (I) entspricht, in der A und Z' beide Wasserstoff sind, X und Y die für die Formel (I) angegebenen Bedeutungen haben, Z Wasserstoff oder Fluoro ist und W ($H,CH_3$), vorzugsweise (H,α-$CH_3$), ist, in der 21-Stellung hydrolysiert wird und, wo erwünscht, ein so erhaltenes 17α,21-Diol-17α-furoat oder -thenoat an C-21 wieder unter Bildung eines anderen geforderten 17α,21-Diesters der allgemeinen Formel (I) verestert wird.

11. Verfahren nach Anspruch 8, Verfahren C, dadurch gekennzeichnet, dass ein 3,20-Dioxo-1,4-pregnadien-21-reaktionsfähiges Acylat, wie etwa das 21-Sulfonat, das ansonsten einem solchen der Formel (I) entspricht, in der A und Z' beide Wasserstoff sind, X Fluoro ist, Y (H,β-OH) ist, Z Wasserstoff oder Fluoro ist, W ($H,CH_3$), vorzugsweise (H,α-$CH_3$), ist und V ein Acylrest der Furan- oder Thiophencarbonsäure ist, an der C-21-Stellung mit einer geeigneten Chloridionenquelle chloriert wird.

12. Verfahren nach Anspruch 8, Verfahren C, dadurch gekennzeichnet, dass zwei Chloratome oder eine 9α-Chloro- und eine 11β-Hydroxygruppe an die 9(11)-Doppelbindung eines 3,20-Dioxo-1,4,9(11)-pregnatriens, das ansonsten einem solchen der Formel (I) entspricht, in der A und Z' beide Wasserstoff sind, Z Wasserstoff oder Fluoro ist, W ($H,CH_3$), vorzugsweise (H,α-$CH_3$), ist, G Chloro, Fluoro oder ein Acyloxyrest ist, wie er in der Definition für $-QV_2$ festgelegt ist, und V ein Acylrest der Furan- oder Thiophencarbonsäure ist, unter Bildung der geforderten 9α,11β-Dichloro- und 9α-Chloro-11β-hydroxyverbindung der allgemeinen Formel (I) addiert werden und, wo erwünscht, eine 21-Acylatfunktion in die entsprechende 21-Chlorofunktion umgewandelt wird.

13. Verfahren nach Anspruch 8, Verfahren C, dadurch gekennzeichnet, dass unter gleichzeitiger Anlagerung von HF oder HCl der 9β,11β-Epoxidring eines 9β,11β-Oxido-3,20-dion-1,4-pregnadiens, das ansonsten einem solchen der Formel (I) entspricht, in der A und Z' beide Wasserstoff sind, Z Wasserstoff oder Fluoro ist, W ($H,CH_3$), vorzugsweise (H,α-$CH_3$), ist, G Chloro,

Fluoro oder ein Acyloxyrest ist, wie er in der Definition für $-QV_2$ festgelegt ist, und V ein Acylrest der Furan- oder Thiophencarbonsäure ist, unter Bildung der geforderten 9α-Chloro-11β-hydroxy- oder 9α-Fluoro-11β-hydroxyverbindung der allgemeinen Formel (I) geöffnet wird.

14. Verfahren nach Anspruch 8, Verfahren D, dadurch gekennzeichnet, dass ein 3,11,20-Trioxo-1,4-pregnadien, das ansonsten einem solchen der Formel (I) entspricht, in der A und Z' beide Wasserstoff sind, X Wasserstoff oder Fluoro ist, Z Wasserstoff oder Fluoro ist, W $(H,CH_3)$, vorzugsweise $(H,\alpha\text{-}CH_3)$, V ein Acylrest der Furan- oder Thiophencarbonsäure ist und G ein Acyloxyrest ist, wie er in der Definition für $-QV_2$ festgelegt ist, in der C-11-Stellung reduziert wird.

15. Verbindung nach irgendeinem der Ansprüche 1 bis 7 zur Verwendung als Pharmazeutikum, insbesondere zur Verwendung als antiinflammatorisches Mittel.

16. Pharmazeutische Zusammensetzung, insbesondere zur Verwendung als antiinflammatorisches Mittel, enthaltend als Wirkstoff eine Verbindung nach irgendeinem der Ansprüche 1 bis 7 zusammen mit einem geeigneten pharmazeutischen Träger.

**Patentansprüche** für den Vertragsstaat: AT

1. Verfahren zur Herstellung von 3,20-Dioxo-1,4-pregnadien-17α-ol-17-aromatisch-heterocyclischen Carboxylaten der allgemeinen Formel (I):

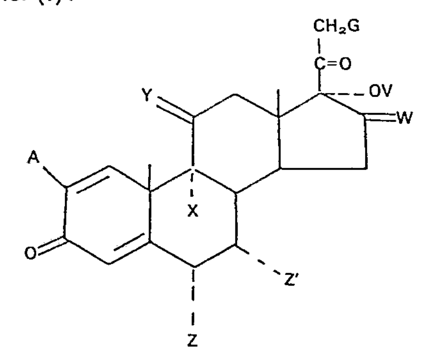

(I)

in der

A Wasserstoff ist oder, vorausgesetzt, dass Y (H,β-OH) ist, auch Chloro, Fluoro oder Methyl sein kann;

X Wasserstoff oder ein Halogenatom mit einem Atomgewicht von weniger als 100 ist;

Y Sauerstoff oder (H,β-OH) ist oder, vorausgesetzt, dass X Wasserstoff ist, auch (H,H) sein kann oder, vorausgesetzt, dass X Chloro oder Bromo ist, auch (H,β-Halogen) sein kann, wobei das β-Halogen ein Atomgewicht von weniger als 100 besitzt und wenigstens so elektronegativ wie X ist;

Z Wasserstoff, Methyl, Chloro oder Fluoro ist;

Z' Wasserstoff ist oder, vorausgesetzt, dass Z Wasserstoff ist, auch Halogen mit einem Atomgewicht von weniger als 100 sein kann;

V ein Acylrest einer Thiophencarbonsäure, Pyrrolcarbonsäure oder Furancarbonsäure oder eines davon abgeleiteten methyl- oder halogensubstituierten Derivats ist;

W (H,H), $(H,C_{1\text{-}6}\text{-Alkyl})$ oder $(H,\alpha OV_1)$ ist, worin $V_1$ Wasserstoff oder ein Acylrest der Retinasäure (Vitamin-A-Säure) oder einer Carbonsäure mit bis zu 12 Kohlenstoffatomen ist, oder W = CHT ist, worin T Wasserstoff, $C_{1\text{-}6}$-Alkyl, Fluoro oder Chloro ist, und

G Wasserstoff, ein Halogenatom mit einem Atomgewicht von weniger als 100 oder $-QV_2$ ist, worin Q ein Sauerstoff- oder Schwefelatom ist und $V_2$ die für V oder $V_1$ angegebene Bedeutung hat oder ein Acylrest der Phosphorsäure ist, der in Form eines Mono- oder Dialkalimetallsalzes oder eines Erdalkalimetallsalzes derselben vorliegen kann,

sowie der 6-Dehydro- und 1,2-Dihydroderivate der vorgenannten Verbindungen, dadurch gekennzeichnet, dass es eines oder mehrere der nachstehenden Verfahren A bis D umfasst:

A: Einführung einer gewünschten Estergruppe in einer oder mehrerer der 16α-, 17α- und/oder 21-Stellungen eines Ausgangsmaterials, das ansonsten einem solchen der Formel (I) entspricht;

B: Hydrolyse einer in einer oder mehrerer der 11β-, 16α- und 21-Stellungen vorhandenen Estergruppe eines Ausgangsmaterials, das ansonsten einem solchen der Formel (I) entspricht;

C: Halogenierung in einer oder mehrerer der Stellungen 9α, 11β und 21 eines 9(11)-En-, eines 9β,11β-Epoxid- oder eines 21-reaktionsfähigen Acylatausgangsmaterials unter Bildung einer entsprechenden 9α,11β-Dihalogeno-, 9α-Halogeno-11β-hydroxy- oder 21-Halogenoverbindung der allgemeinen Formel (I), und

D: Reduktion der 11-Ketogruppe eines 11-Onausgangsmaterials zu einer entsprechenden 11β-Hydroxyverbindung der allgemeinen Formel (I).

2. Verfahren nach Anspruch 1, Verfahren A, dadurch gekennzeichnet, dass ein 3,20-Dioxo-1,4-pregnadien-17α,21-diol-21-acylat, das ansonsten einem solchen der Formel (I) entspricht, in der A und Z' beide Wasserstoff sind, X Chloro ist und Y (H,β-Cl) ist, oder X Fluoro ist und Y (H,β-OH) ist, Z Wasserstoff oder Fluoro ist, W $(H,CH_3)$, vorzugsweise $(H,\alpha\text{-}CH_3)$, ist und G ein Acyloxyrest ist, wie er in der Definition für $-QV_2$ festgelegt ist, mit einem reaktionsfähigen Derivat der Furan- oder Thiophencarbonsäure verestert wird.

3. Verfahren nach Anspruch 1, Verfahren B, dadurch gekennzeichnet, dass ein 17α-Furoat-21-acylat oder 17α-Thenoat-21-acylat oder ein 17α,21-Orthoester, das heisst, ein 17α,21-Alkylorthofuroat oder -thenoat, das ansonsten einem solchen der Formel (I) entspricht, in der A und Z' beide Wasserstoff sind, X und Y die für die Formel (I) angegebenen Bedeutungen haben, Z Wasserstoff oder Fluoro ist und W $(H,CH_3)$, vorzugsweise $(H,\alpha\text{-}CH_3)$, ist, in der 21-Stellung hydrolysiert wird und, wo erwünscht, ein so erhaltenes 17α,21-Diol-17α-furoat oder -thenoat an C-21 wieder unter Bildung eines anderen geforderten 17α,21-Diesters der allgemeinen Formel (I) verestert wird.

4. Verfahren nach Anspruch 1, Verfahren C, dadurch gekennzeichnet, dass ein 3,20-Dioxo-1,4-pregnadien-21-reaktionsfähiges Acylat, wie etwa das 21-Sulfonat, das ansonsten einem solchen der Formel (I) entspricht, in der A und Z' beide Wasserstoff sind, X Fluoro ist, Y (H,β-OH) ist, Z Wasserstoff oder Fluoro ist, W ($H,CH_3$), vorzugsweise ($H,\alpha$-$CH_3$), ist und V ein Acylrest der Furan- oder Thiophencarbonsäure ist, an der C-21-Stellung mit einer geeigneten Chloridionenquelle chloriert wird.

5. Verfahren nach Anspruch 1, Verfahren C, dadurch gekennzeichnet, dass zwei Chloratome oder eine 9α-Chloro- und eine 11β-Hydroxygruppe an die 9(11)-Doppelbindung eines 3,20-Dioxo-1,4,9(11)-pregnatriens, das ansonsten einem solchen der Formel (I) entspricht, in der A und Z' beide Wasserstoff sind, Z Wasserstoff oder Fluoro ist, W ($H,CH_3$), vorzugsweise ($H,\alpha$-$CH_3$), ist, G Chloro, Fluoro oder ein Acyloxyrest ist, wie er in der Definition für $-QV_2$ festgelegt ist, und V ein Acylrest der Furan- oder Thiophencarbonsäure ist, unter Bildung der geforderten 9α,11β-Dichloro- und 9α-Chloro-11β-hydroxyverbindung der allgemeinen Formel (I) addiert werden und, wo erwünscht, eine 21-Acylatfunktion in die entsprechende 21-Chlorofunktion umgewandelt wird.

6. Verfahren nach Anspruch 1, Verfahren C, dadurch gekennzeichnet, dass unter gleichzeitiger Anlagerung von HF oder HCl der 9β,11β-Epoxidring eines 9β,11β-Oxido-3,20-dion-1,4-pregnadiens, das ansonsten einem solchen der Formel (I) entspricht, in der A und Z' beide Wasserstoff sind, Z Wasserstoff oder Fluoro ist, W ($H, CH_3$), vorzugsweise ($H,\alpha$-$CH_3$), ist, G Chloro, Fluoro oder ein Acyloxyrest ist, wie er in der Definition für $-QV_2$ festgelegt ist, und V ein Acylrest der Furan- oder Thiophencarbonsäure ist, unter Bildung der geforderten 9α-Chloro-11β-hydroxy- oder 9α-Fluoro-11β-hydroxyverbindung der allgemeinen Formel (I) geöffnet wird.

7. Verfahren nach Anspruch 1, Verfahren D, dadurch gekennzeichnet, dass ein 3,11,20-Trioxo-1,4-pregnadien, das ansonsten einem solchen der Formel (I) entspricht, in der A und Z' beide Wasserstoff sind, X Wasserstoff oder Fluoro ist, Z Wasserstoff oder Fluoro ist, W ($H,CH_3$), vorzugsweise ($H,\alpha$-$CH_3$), V ein Acylrest der Furan- oder Thiophencarbonsäure ist und G ein Acyloxyrest ist, wie er in der Definition für $-QV_2$ festgelegt ist, in der C-11-Stellung reduziert wird.

8. Verfahren nach Anspruch 1, Verfahren B, dadurch gekennzeichnet, dass ein 11β-geschütztes Hydroxy-17α-furoat- (oder -17α-thenoat-)-21-acylat, das ansonsten einem solchen der Formel (I) entspricht, in der A und Z' beide Wasserstoff sind, X Halogen, vorzugsweise Fluoro, ist, Z Wasserstoff oder Fluoro ist und W ($H,CH_3$), vorzugsweise ($H,\alpha$-$CH_3$), ist und das 21-Acylat die Gruppe $-QV_2$ ist, in der Q und $V_2$ die für die Formel (I) angegebenen Bedeutungen haben, unter Bildung des entsprechenden 11β-freien Hydroxy-17α,21-diesters der allgemeinen Formel (I) in der 11-Stellung hydrolysiert wird.

9. Verfahren zur Herstellung einer pharmazeutischen Zusammensetzung, dadurch gekennzeichnet, dass eine Verbindung der in Anspruch 1 angegebenen allgemeinen Formel (I) mit einem geeigneten, pharmazeutisch unbedenklichen Träger vermischt wird.

10. Verfahren nach Anspruch 9, dadurch gekennzeichnet, dass die Verbindung der in Anspruch 1 angegebenen allgemeinen Formel (I) mittels eines der in irgendeinem der Ansprüche 1 bis 8 beanspruchten Verfahren hergestellt ist.

**Revendications** pour les Etats contractants BE, CH, DE, FR, GB, IT, LI, NL, SE

1. Carboxylates 3,20-dioxo-1,4-prégnadiène-17α-ol 17-aromatiques hétérocycliques de formule générale (I):

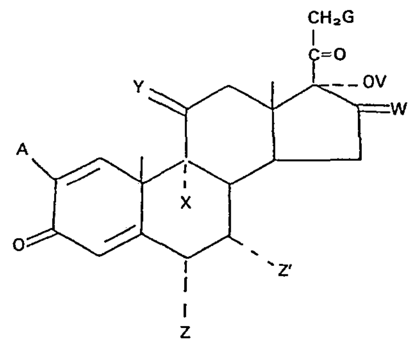

(I)

caractérisés en ce que A est de l'hydrogène ou, à condition que Y soit (H, β-OH), A peut également être chloro, fluoro ou méthyle;

X est de l'hydrogène ou un atome d'halogène ayant un poids atomique inférieur à 100;

Y est de l'oxygène, (H,β-OH) ou bien, à condition que X soit de l'hydrogène, Y peut également être (H,H) ou, à condition que X soit chloro ou bromo, Y peut également être (H, β-halogène), le β-halogène ayant un poids atomique inférieur à 100 et étant au moins aussi électronégatif que X;

Z est de l'hydrogène, du méthyle, chloro ou fluoro;

Z' est de l'hydrogène ou, à condition que Z soit de l'hydrogène, peut également être un halogène ayant un poids atomique de moins de 100;

V est un radical acyle d'un acide thiophènecarboxylique, d'un acide pyrrolecarboxylique ou d'un acide furannecarboxylique ou bien les dérivés substitués en méthyle ou en halogène;

W est (H,H), (H, alcoyle $C_1$-$C_6$) ou ($H, \alpha OV_1$) où $V_1$ est de l'hydrogène ou un radical acyle de l'acide rétinoïque ou bien un acide carboxylique ayant jusqu'à 12 atomes de carbone ou bien W est =CHT où T est de l'hydrogène, un alcoyle $C_1$-$C_6$, fluoro ou chloro, et

G est de l'hydrogène, un atome d'halogène ayant un poids atomique inférieur à 100 ou $-QV_2$ où Q est un atome d'oxygène ou de soufre et $V_2$ est tel que défini pour V ou $V_1$ ou est un radical acyle de l'acide phosphorique qui peut avoir la forme d'un sel d'un métal mono- ou dialcalin ou d'un métal alcalino-terreux,

et les dérivés 6-déhydro et 1,2-dihydro de ce qui précède.

2. Composé selon la revendication 1, caractérisé en ce qu'il est un 3,20-dioxo-1,4-prégnadiène.

3. Composé selon l'une des revendications 1 ou 2, caractérisé en ce que V est furannecarbonyle ou thiophènecarbonyle.

4. Composé selon l'une des revendications 1 à 3, caractérisé en ce qu'il a pour formule générale (II):

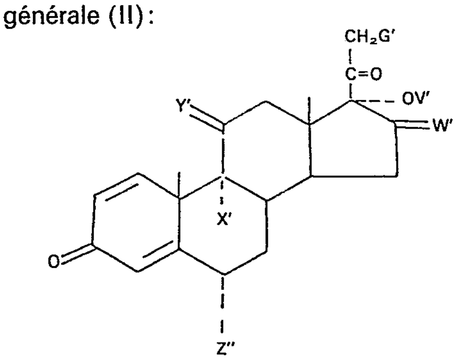

(II)

où X' est fluoro ou chloro;

Y' est (H,β-OH) ou, à condition que X' soit chloro, Y' peut également être (H,β-halogène), le β-halogène ayant un poids atomique de moins de 100 et étant au moins aussi électronégatif que X';

Z'' est hydrogène ou fluoro;

W' est (H,H) ou (H,$CH_3$);

V' est furannecarbonyle ou thiophènecarbonyle, et

G' est chloro ou fluoro ou bien $-QV'_2$ où Q est un atome d'oxygène ou de soufre (de préférence de l'oxygène) et $V'_2$ est de l'hydrogène ou un radical acyle de l'acide rétinoïque, d'un acide carboxylique ayant jusqu'à 12 (de préférence jusqu'à 8) atomes de carbone ou de l'acide phosphorique qui peut avoir la forme d'un sel d'un métal mono- ou dialcalin ou alcalino-terreux.

5. Composé selon la revendication 4, caractérisé en ce que G' est chloro, alcanoyloxy $C_1$-$C_6$ ou alcoxy $C_1$-$C_6$-alcanoyloxy $C_1$-$C_6$ (de préférence acétoxy ou méthoxyacétoxy), X' est chloro et Y' est (H,β-Cl) ou X' est chloro ou fluoro et Y' est (H,β-OH), et W' est (H,H) ou (H,$CH_3$), de préférence (H,α-$CH_3$).

6. Composé selon la revendication 1, caractérisé en ce qu'il est choisi parmi:

9α,11β,21-trichloro-16α-méthyl-1,4-prégnadiène-17α-ol-3,20-dione 17-(2'-furoate);

6α-fluoro-9α,11β,21-trichloro-16α-méthyl-1,4-prégnadiène-17α-ol-3,20-dione 17-(2'-furoate);

9α-fluoro-16α-méthyl-1,4-prégnadiène-11β,17α,21-triol-3,20-dione 17-(2'-furoate) 21-acétate;

9α-fluoro-16α-méthyl-1,4-prégnadiène-11β,17α,21-triol-3,20-dione 17-(2'-furoate) 21-méthoxyacétate;

9α-fluoro-16α-méthyl-1,4-prégnadiène-11β,17α,21-triol-3,20-dione 17-(3'-furoate) 21-acétate;

9α-fluoro-21-chloro-16α-méthyl-1,4-prégnadiène-11β,17α-diol-3,20-dione 17-(2'-furoate);

9α,21-dichloro-16α-méthyl-1,4-prégnadiène-11β,17α-diol-3,20-dione 17-(2'-furoate);

9α-chloro-16α-méthyl-1,4-prégnadiène-11β,17α,21-triol-3,20-dione 17-(2'-furoate) 21-acétate;

9α-chloro-16α-méthyl-1,4-prégnadiène-11β,17α,21-triol-3,20-dione 17-(2'-furoate) 21-méthoxyacétate;

9α-fluoro-21-chloro-16α-méthyl-1,4-prégnadiène-11β,17α-diol-3,20-dione 17-(2'-furoate), et

9α-chloro-16α-méthyl-1,4-prégnadiène-11β,17α-diol-21-thiol-3,20-dione 17-(2'-furoate) 21-pivalate.

7. Composé selon la revendication 1, ledit composé étant choisi parmi:

9α,11β,21-trichloro-16α-méthyl-1,4-prégnadiène-17α-ol-3,20-dione 17-(2'-thénoate);

9α-fluoro-16α-méthyl-1,4-prégnadiène-11β,17α,21-triol-3,20-dione 17-(2'-thénoate) 21-acétate;

9α-fluoro-21-chloro-16α-méthyl-1,4-prégnadiène-11β,17α-diol-3,20-dione 17-(2'-thénoate), et

9α,21-dichloro-16α-méthyl-1,4-prégnadiène-11β,17α-diol-3,20-dione 17-(2'-thénoate).

8. Procédé de préparation d'un carboxylate 17-aromatique hétérocyclique selon la revendication 1, caractérisé en ce qu'une matière première appropriée de 3,20-dioxo-1,4-prégnadiène ou son dérivé 6-déhydro ou 1,2-dihydro est soumis à:

A: introduction du groupe ester souhaité aux positions 16α, 17α et/ou 21, ou

B: hydrolyse d'un groupe ester présent à une ou plusieurs des positions 11β, 16α et 21 ou d'un groupe 16α, 17α- ou 17α,21-orthoester, ou

C: halogénation en une ou plusieurs des positions 9α, 11β et 21, ou

D: réduction d'un groupe 11-oxo en un groupe 11β-hydroxy.

9. Procédé selon la revendication 8, procédé A, caractérisé par l'estérification d'un 3,20-dioxo-1,4-prégnadiène-17α,21-diol 21-acylate autrement correspondant à celui de formule (I) où A et Z' sont tous deux de l'hydrogène, X est chloro et Y est (H,β-Cl) ou X est fluoro et Y est (H,β-OH), Z est hydrogène ou fluoro, W est (H,$CH_3$), de préférence (H,α-$CH_3$), et G est un radical acyloxy tel que défini dans la définition de $-QV_2$, avec un dérivé réactif d'acide furanne- ou thiophènecarboxylique.

10. Procédé selon la revendication 8, procédé B, caractérisé par l'hydrolyse à la position 21 d'un 17α-furoate 21-acylate ou 17α-thénoate 21-acylate ou d'un 17α,21-orthoester, c'est-à-dire un 17α,21-alkylorthofuroate ou -thénoate autrement correspondant à celui de formule (I) où A et Z' sont tous deux de l'hydrogène, X et Y sont tels que définis pour la formule (I), Z est de l'hydrogène ou fluoro et W est (H,$CH_3$), de préférence (H,α-$CH_3$), et, si on le souhaite, la réestérification en C-21 d'un 17α,21-diol 17α-furoate ou -thénoate ainsi obtenu pour donner un autre 17α,21-diester requis de formule générale (I).

11. Procédé selon la revendication 8, procédé

C, caractérisé par la chloration à la position C-21 d'un 3,20-dioxo-1,4-prégnadiène acylate réactif en position 21, tel qu'un 21-sulfonate, autrement correspondant à celui de la formule générale (I) où A et Z' sont tous deux de l'hydrogène, X est fluoro, Y est (H,β-OH), Z est de l'hydrogène ou fluoro, W est (H, $CH_3$), de préférence (H,α-$CH_3$), et V est un radical acyle de l'acide furanne- ou thiophènecarboxylique, avec une source appropriée de l'ion chlorure.

12. Procédé selon la revendication 8, procédé C, caractérisé par l'addition de deux atomes de chlore ou d'un groupe 9α-chloro et 11β-hydroxy à la double liaison 9(11) d'un 3,20-dioxo-1,4,9(11)-prégnatriène autrement correspondant à celui de formule (I) où A et Z' sont tous deux de l'hydrogène, Z est de l'hydrogène ou fluoro, W est (H,$CH_3$), de préférence (H,α-$CH_3$), G est chloro, fluoro ou un radical acyloxy tel que défini dans la définition de $-QV_2$, et V est un radical acyle de l'acide furanne- ou thiophènecarboxylique pour donner respectivement les composés 9α,11β-dichloro- et 9α-chloro-11β-hydroxy de formule générale (I) et, si on le souhaite, la conversion d'une fonction 21-acylate en fonction 21-chloro correspondante.

13. Procédé selon la revendication 8, procédé C, caractérisé par la décyclisation du noyau 9β,11β-époxyde, avec addition concomitante de HF ou HCl, d'un 9β,11β,oxydo-3,20-dione-1,4-prégnadiène autrement correspondant à celui de formule (I) où A et Z' sont tous deux de l'hydrogène, Z est l'hydrogène ou fluoro, W est (H,$CH_3$), de préférence (H,α-$CH_3$), G est chloro, fluoro ou un radical acyloxy tel que défini dans la définition de $-QV_2$, et V est un radical acyle de l'acide furanne- ou thiophènecarboxylique pour donner respectivement le composé requis 9α-chloro-11β-hydroxy ou 9α-fluoro-11β-hydroxy de formule générale (I).

14. Procédé selon la revendication 8, procédé D, caractérisé par la réduction, à la position C-11, d'un 3,11,20-trioxo-1,4-prégnadiène correspondant à celui de la formule générale (I) où A et Z' sont tous deux de l'hydrogène, X est hydrogène ou fluoro, Z est hydrogène ou fluoro, W est (H,$CH_3$), de préférence (H,α-$CH_3$), V est un radical acyle de l'acide furanne- ou thiophènecarboxylique, et G est un radical acyloxy tel que défini dans la définition de $-QV_2$.

15. Composé selon l'une des revendications 1 à 7, caractérisé en ce qu'il a une utilisation pharmaceutique, en particulier comme agent anti-inflammatoire.

16. Composition pharmaceutique, en particulier pour une utilisation comme agent anti-inflammatoire, caractérisée en ce qu'elle contient comme ingrédient actif un composé selon l'une des revendications 1 à 7 avec un véhicule pharmaceutique approprié.

**Revendications** pour l'Etat contractant: AT

1. Procédé de préparation de carboxylates 3,20-dioxo-1,4-prégnadiène-17α-ol 17-aromatiques hétérocycliques de formule générale (I):

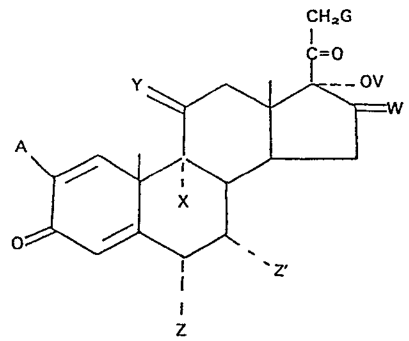

(I)

dans laquelle

A est de l'hydrogène ou, à condition que Y soit (H,β-OH), A peut également être chloro, fluoro ou méthyle;

X est de l'hydrogène ou un atome d'halogène ayant un poids atomique inférieur à 100;

Y est de l'oxygène, (H,β-OH) ou bien, à condition que X soit de l'hydrogène, Y peut également être (H,H) ou, à condition que X soit chloro ou bromo, Y peut également être (H,β-halogène), le β-halogène ayant un poids atomique inférieur à 100 et étant au moins aussi électronégatif que X;

Z est de l'hydrogène, du méthyle, chloro ou fluoro;

Z' est de l'hydrogène ou, à condition que Z soit de l'hydrogène, peut également être un halogène ayant un poids atomique de moins de 100;

V est un radical acyle de l'acide thiophènecarboxylique, de l'acide pyrrolecarboxylique ou de l'acide furannecarboxylique ou bien les dérivés substitués en méthyle ou en halogène;

W est (H,H), (H, alcoyle $C_1$-$C_6$) ou (H,α$OV_1$) où $V_1$ est de l'hydrogène ou un radical acyle de l'acide rétinoïque ou bien un acide carboxylique ayant jusqu'à 12 atomes de carbone ou bien W est =CHT où T est de l'hydrogène, un alcoyle $C_1$-$C_6$, fluoro ou chloro, et

G est de l'hydrogène, un atome d'halogène ayant un poids atomique inférieur à 100 ou $-QV_2$ où Q est un atome d'oxygène ou de soufre et $V_2$ est tel que défini pour V ou $V_1$ ou est un radical acyle de l'acide phosphorique qui peut avoir la forme d'un sel d'un métal mono- ou dialcalin ou d'un métal alcalino-terreux,

et les dérivés 6-déhydro et 1,2-dihydro de ce qui précède, caractérisé en ce qu'il comprend un ou plusieurs des procédés A à D qui suivent:

A: introduction d'un groupe ester souhaité en une ou plusieurs des positions 16α, 17α et/ou 21 d'une matière première autrement correspondant à celle de la formule générale (I);

B: hydrolyse d'un groupe ester présent en une ou plusieurs des positions 11β, 16α et 21 dans une matière première autrement correspondant à celle de la formule générale (I);

C: halogénation en une ou plusieurs positions 9α, 11β et 21 d'un 9(11)-ène, un 9β,11β-époxyde ou un acylate réactif en position 21 comme matière première pour donner un composé correspondant

9α,11β-dihalo, 9α-halo-11β-hydroxy ou 21-halo de formule générale (I), et

D: réduction au groupe 11-céto d'une matière première 11-one pour donner un composé 11β-hydroxy correspondant de formule générale (I).

2. Procédé selon la revendication 1, procédé A, caractérisé par l'estérification d'un 3,20-dioxo-1,4-prégnadiène 17α,21-diol 21-acylate autrement correspondant à celui de formule (I) où A et Z' sont tous deux de l'hydrogène, X est chloro et Y est (H,β-Cl) ou X est fluoro et Y est (H,β-OH), Z est hydrogène ou fluoro, W est $(H,CH_3)$, $(H,\alpha\text{-}CH_3)$, et G est un radical acyloxy tel que défini dans la définition de $-QV_2$ avec un dérivé réactif de l'acide furanne- ou thiophènecarboxylique.

3. Procédé selon la revendication 1, procédé B, caractérisé par l'hydrolyse à la position 21 d'un 17α-furoate 21-acylate ou 17α-thénoate 21-acylate ou d'un 17α,21-orthoester qui est un 17α,21-alkylorthofuroate ou -thénoate, autrement correspondant à celui de formule (I) où A et Z' sont tous deux de l'hydrogène, X et Y sont tels que définis pour la formule (I), Z est hydrogène ou fluoro et W est $(H,CH_3)$, de préférence $(H,\alpha\text{-}CH_3)$, et, si on le souhaite, la réestérification en C-21 d'un 17α,21-diol 17α-furoate ou -thénoate ainsi obtenu pour donner un autre 17α,21-diester requis de formule générale (I).

4. Procédé selon la revendication 1, procédé C, caractérisé par une chloruration à la position C-21 d'un 3,20-dioxo-1,4-prégnadiène 21-acylate réactif, comme le 21-sulfonate, correspondant autrement à celui de formule générale (I) où A et Z' sont tous deux de l'hydrogène, X est fluoro, Y est (H,β-OH), Z est de l'hydrogène ou fluoro, W est $(H,CH_3)$, de préférence $(H,\alpha\text{-}CH_3)$, et V est un radical acyle de l'acide furanne- ou thiophènecarboxylique avec une source appropriée d'un ion de chlore.

5. Procédé selon la revendication 1, procédé C, caractérisé par l'addition de deux atomes de chlore ou d'un groupe 9α-chloro et 11β-hydroxy, à la double liaison 9(11) d'un 3,20-dioxo-1,4,9(11)-prégnatriène correspondant autrement à celui de la formule générale (I) où A et Z' sont tous deux de l'hydrogène, Z est hydrogène ou fluoro, W est $(H,CH_3)$, de préférence $(H,\alpha\text{-}CH_3)$, G est chloro, fluoro ou un radical acyloxy tel que défini à la définition de $-QV_2$ et V est un radical acyle de l'acide furanne- ou thiophènecarboxylique pour donner les composés 9α,11β-dichloro et 9α-chloro-11β-hydroxy requis de formule générale (I), et, si on le souhaite, la conversion d'une fonction 21-acylate en fonction 21-chloro correspondante.

6. Procédé selon la revendication 1, procédé C, caractérisé par une cyclisation du noyau 9β,11β-époxyde, avec addition concomitante de HF ou HCl d'un 9β,11β-oxydo-3,20-dione-1,4-prégnadiène autrement correspondant à celui de la formule générale (I) où A et Z' sont tous deux de l'hydrogène, Z est de l'hydrogène ou fluoro, W est $(H,CH_3)$, de préférence $(H,\alpha\text{-}CH_3)$, G est chloro, fluoro ou un radical acyloxy tel que défini dans la définition de $-QV_2$ et V est un radical acyle de l'acide furanne- ou thiophènecarboxylique pour donner le composé 9α-chloro-11β-hydroxy ou 9α-fluoro-11β-hydroxy de formule générale (I).

7. Procédé selon la revendication 1, procédé D, caractérisé par la réduction à la position C-11 d'un 3,11,20-trioxo-1,4-prégnadiène correspondant à celui de la formule générale (I) où A et Z' sont tous deux de l'hydrogène, X est hydrogène ou fluoro, Z est hydrogène ou fluoro, W est $(H,CH_3)$, de préférence $(H,\alpha\text{-}CH_3)$, V est un radical acyle de l'acide furanne- ou thiophènecarboxylique et G est un radical acyloxy tel que défini à la définition de $-QV_2$.

8. Procédé selon la revendication 1, procédé B, caractérisé par l'hydrolyse à la position 11 d'un hydroxy 17α-furoate (ou 17α-thénoate) 21-acylate protégé en position 11β autrement correspondant à celui de la formule générale (I) où A et Z' sont tous deux de l'hydrogène, X est un halogène, de préférence fluoro, Z est hydrogène ou fluoro, W est $(H,CH_3)$, de préférence $(H,\alpha\text{-}CH_3)$, et le 21-acylate est le groupe $-QV_2$ où Q et $V_2$ sont tels que définis pour la formule générale (I) pour donner l'hydroxy-17α,21-diester libre en 11β de formule générale (I).

9. Procédé de préparation d'une composition pharmaceutique qui consiste à mélanger un composé de formule générale (I) indiqué à la revendication 1 avec un véhicule acceptable en pharmacie approprié.

10. Procédé selon la revendication 9, où le composé de formule générale (I) indiqué à la revendication 1 a été préparé par un procédé selon l'une des revendications 1 à 8.